# FASCICULE DE BREVET EUROPEEN

(11) **EP 4 090 334 B1**
(45) Date de publication et mention de la délivrance du brevet: **16.07.2025**
(21) Numéro de dépôt: 21704870.1
(22) Date de dépôt: 12.01.2021
(51) Int. Cl.: A61K 31/554, A61K 31/5383, A61K 31/506, A61K 31/53, A61K 45/06, A61P 31/12, A61P 31/16, A61K 9/00

(54) **COMBINAISON DE DILTIAZEM ET DE BALOXAVIR MARBOXIL POUR SON UTILISATION DANS LA PRÉVENTION ET/OU THE TRAITEMENT D'UNE INFECTION VIRALE PAR UN VIRUS INFLUENZA**
KOMBINATION AUS DILTIAZEM UND BALOXAVIR MARBOXIL ZUR VORBEUGUNG UND/ODER BEHANDLUNG EINER VIRALEN ANSTECKUNG DURCH DAS INFLUENZA-VIRUS
COMBINATION OF DILTIAZEM AND BALOXAVIR MARBOXIL FOR USE IN THE PREVENTION AND/OR TREATMENT OF A VIRAL INFECTION BY THE INFLUENZA VIRUS

(30) Priorité: 13.01.2020 FR 2000255
(43) Date de publication de la demande: 23.11.2022
(73) Titulaire: Université Claude Bernard Lyon 1, 69100 Villeurbanne (FR); Institut National de la Santé et de la Recherche Médicale (INSERM), 75013 Paris (FR); Centre National de la Recherche Scientifique (CNRS), 75016 Paris (FR); Université Laval, Québec, Québec G1V 0A6 (CA); Ecole Normale Supérieure de Lyon, 69007 Lyon (FR)
(72) Inventeur: TERRIER, Olivier, 69008 LYON (FR); ROSA-CALATRAVA, Manuel, 69003 LYON (FR); BOIVIN, Guy, QUEBEC, QUEBEC G1V1E7 (CA); PIZZORNO, Mario-Andres, 69007 LYON (FR); PADEY, Blandine, 69005 LYON (FR)
(74) Mandataire: Regimbeau
(86) Numéro de dépôt international: PCT/FR2021/050049
(87) Numéro de publication internationale: WO 2021/144528

(56) Documents cités:
- WO-A2-2016/146836
- ANDRÉS PIZZORNO ET AL: "Repurposing of Drugs as Novel Influenza Inhibitors From Clinical Gene Expression Infection Signatures", FRONTIERS IN IMMUNOLOGY, vol. 10, 1 January 2019 (2019-01-01), pages 60, XP055739937, DOI: 10.3389/fimmu.2019.00060
- KEITA FUKAO ET AL: "Combination treatment with the cap-dependent endonuclease inhibitor baloxavir marboxil and a neuraminidase inhibitor in a mouse model of influenza A virus infection", JOURNAL OF ANTIMICROBIAL CHEMOTHERAPY., vol. 74, no. 3, 1 March 2019 (2019-03-01), GB, pages 654 - 662, XP055700407, ISSN: 0305-7453, DOI: 10.1093/jac/dky462
- ANDRÉS PIZZORNO ET AL: "Drug Repurposing Approaches for the Treatment of Influenza Viral Infection: Reviving Old Drugs to Fight Against a Long-Lived Enemy", FRONTIERS IN IMMUNOLOGY, vol. 10, 10 March 2019 (2019-03-10), pages 531, XP055739932, DOI: 10.3389/fimmu.2019.00531

## Description

### DOMAINE DE L'INVENTION

La présente invention concerne une nouvelle combinaison de diltiazem et de baloxavir marboxil, pour son utilisation dans la prévention et/ou le traitement d'une infection virale par le virus influenza. La présente invention concerne également un produit de combinaison comprenant du diltiazem et du baloxavir marboxil, pour son utilisation simultanée, séparée ou séquentielle dans la prévention et/ou le traitement d'une infection virale par un virus influenza.

### ETAT DE LA TECHNIQUE

Les infections respiratoires aigües (IRA) humaines représentent une des causes principales de consultations, d'hospitalisations et de décès dans le monde, en étant notamment la première cause de mortalité chez les jeunes enfants avec près de 2 millions de décès par an.

Parmi les agents étiologiques responsables des infections respiratoires aigües, les virus occupent une place prépondérante. Ils sont en effet retrouvés dans la majorité des cas de pneumonies infantiles et sont un facteur prédisposant des pneumonies bactériennes chez l'adulte.

Parmi les virus les plus représentatifs en termes de fréquence (épidémies saisonnières) et de morbidité, les virus influenza de type A et B sont prévalents et constituent de plus un facteur de risque pandémique récurrent. Les virus influenza constituent également des agents étiologiques contribuant à une co-morbidité et une co-mortalité accrues dans le cas des co-infections respiratoires et contribuent aussi à l'émergence de souches de bactéries résistantes aux antibiotiques, qui remettent fondamentalement en question l'efficacité des traitements antibiotiques tant chez l'Homme que chez l'animal.

En termes de traitements prophylactiques ou thérapeutiques contre les virus, notamment contre les virus influenza, l'arsenal actuel est plutôt limité.

A ce jour, la vaccination est la stratégie principale de la prévention de la grippe. Toutefois, l'utilisation des vaccins présente des inconvénients. Par exemple, lors de l'apparition d'une nouvelle souche virale saisonnière, un délai de 6 à 9 mois est nécessaire pour la mise au point et la délivrance d'un nouveau vaccin. De plus, on constate une inadéquation des vaccins lorsque les souches circulantes mutent. Enfin, il est difficile voire impossible de produire des vaccins contre certaines souches pandémiques d'origine aviaire telles que H5N1 ou H7N9, par le processus classique de production vaccinale basé sur l'œuf de poule embryonné.

La revue (Pizzorno *et al.,* 2019b) présente les principaux composés thérapeutiques utilisés dans le cadre du traitement thérapeutique de la grippe, ainsi que leurs modes d'action.

Lorsqu'une action thérapeutique est nécessaire, les principaux composés antiviraux actuellement disponibles sont les suivants : Oseltamivir/Tamiflu^{®} (ROCHE) ; Zanamivir/Relenza^{®} (GSK) ; et Baloxavir Marboxil/Xofluza^{®} (ROCHE) qui est disponible dans quelques pays dont les États-Unis et le Japon.

Le Zanamivir et l'Oseltamivir sont des inhibiteurs de la neuraminidase.

Le Baloxavir marboxil est un inhibiteur du complexe polymérase des virus influenza ; il a également été récemment caractérisé comme un inhibiteur de polymérases d'autres virus à ARN génomique de polarité négative (Wang *et al.,* 2020).

Bien que relativement efficaces, ces traitements antiviraux présentent également des inconvénients significatifs : en premier lieu l'émergence croissante de virus résistants auxdits composés antiviraux, et en second lieu l'absence d'activité antivirale à large spectre de ces composés.

En effet, ces composés antiviraux ciblent les déterminants viraux et non la cellule hôte. Du fait de la fréquence élevée de mutations des virus influenza (inhérente à leur polymérase peu fidèle en termes de réplication et à leur génome segmenté et source de réassortiments génétiques entre différents virus), les antiviraux classiques induisent des résistances virales récurrentes.

Dans le cas du Baloxavir marboxil, plusieurs études récentes indiquent que le risque de développer une résistance est très élevé. Dans une étude clinique de phase 3, l'incidence de souches résistantes était de près de 10%, principalement chez les virus A(H3N2). De façon plus préoccupante, le taux de résistance s'élevait à près de 20% dans une étude pédiatrique (Omoto et al., 2018). En outre, une étude récente complémentaire sur les mutants de résistance au Baloxavir marboxil a montré qu'une des mutations principales de résistance (PA I38T) entraine un délai d'apparition de la réduction des symptômes et une prolongation de l'excrétion virale (Uehara *et al.,* 2019).

Enfin, certains patients infectés non-traités au Baloxavir marboxil ont excrété une souche résistante, ce qui témoigne d'une bonne transmissibilité entre individus des virus résistants au Baloxavir et fait craindre une possible dissémination à grande échelle de ces virus mutants (Takashita *et al.,* 2019). Or, l'impact clinique de la résistance s'avère particulièrement problématique chez les sujets hospitalisés et chez les individus immunodéprimés, entre autres.

De nouveaux composés antiviraux sont ainsi activement recherchés, dans le but de limiter l'apparition de ces résistances. En particulier, les thérapies de combinaison basées sur l'association d'au moins deux composés antiviraux semblent particulièrement prometteuses.

Notamment, lorsque deux composés antiviraux combinés présentent une synergie d'action, cela permet de diminuer les doses de composés actifs utilisés et ainsi de limiter les risques d'apparition de résistances tout en assurant un effet thérapeutique antiviral significatif.

Parmi les nouveaux composés antiviraux identifiés ces dernières années, on citera notamment le Diltiazem, qui est un composé actif qui agit sur la cellule hôte plutôt que sur le virus, permettant ainsi de traiter différentes infections virales.

La demande internationale WO 87/07508 décrit l'utilisation du Diltiazem pour le traitement d'infections virales liées au cytomégalovirus ou à l'herpès.

La demande internationale WO 2011/066657 décrit l'utilisation du Diltiazem pour le traitement ou la prévention d'infections virales telles que l'herpès buccal, l'herpès génital et le zona.

La demande internationale WO 2016/146836 ainsi que l'article (Pizzorno *et al.,* 2019a) décrivent l'utilisation du Diltiazem pour traiter les infections par les virus influenza, éventuellement en combinaison avec d'autres agents antiviraux. Une combinaison de Diltiazem et d'Oseltamivir est proposée pour traiter les infections virales par influenza.

La demande internationale WO 2019/224489 expose en détails certains des effets biologiques du Diltiazem, qui induit l'expression des gènes codant pour les interférons de type III dans les cellules de l'épithélium respiratoire. Le Diltiazem peut ainsi être utilisé dans diverses applications thérapeutiques, notamment pour le traitement des infections respiratoires virales et bactériennes dans les épithéliums respiratoires et intestinaux.

Ainsi, le Diltiazem représente une nouvelle option thérapeutique très prometteuse pour le traitement des infections virales.

Toutefois, les nombreux avantages de l'utilisation thérapeutique de ce composé n'ont pas encore été identifiés. En particulier, son utilisation en combinaison avec d'autres agents antiviraux peut présenter des avantages surprenants, qui n'étaient pas prévisibles.

### EXPOSE DE L'INVENTION

La présente invention est relative à une combinaison de Diltiazem et de Baloxavir marboxil pour son utilisation dans la prévention et/ou le traitement d'une infection virale par un virus influenza.

Enfin, l'invention concerne un produit de combinaison comprenant du Diltiazem et du Baloxavir marboxil pour son utilisation simultanée, séparée ou séquentielle dans la prévention et/ou le traitement d'une infection virale par un virus influenza.

### DESCRIPTION DES FIGURES

Dans les figures et leurs légendes, dans un but de simplification, les appellations et abréviations suivantes sont utilisées :
- Dil ou DIL désignent le Diltiazem ;
- FAV désigne le favipiravir ;
- BLX, Bal ou Baloxavir désignent la forme active du Baloxavir (acide de Baloxavir).

La figure 1 représente les effets antiviraux de la combinaison Diltiazem + Baloxavir sur des cellules en culture infectées par le virus H1N1nLuc.
Figure 1a : chronogramme de l'expérience.
Figure 1b : Courbes d'effet-dose des molécules Diltiazem et Baloxavir en monothérapie sur le virus H1N1nLuc (détermination des EC50).
Figure 1c : Représentation graphique des effets synergiques, selon les concentrations utilisées du Diltiazem (DIL) et du Baloxavir (BLX), en combinaison contre un virus recombinant H1N1nLuc.
La figure 2 représente les effets antiviraux de la combinaison Diltiazem + Favipiravir sur des cellules en culture infectées par le virus H1N1nLuc.
Figure 2a : chronogramme de l'expérience
Figure 2b : Courbes d'effet-dose du Diltiazem et Favipiravir en monothérapie sur le virus H1N1nLuc (détermination des EC50).
Figure 2c : Représentation graphique des effets synergiques ou antagonistes, selon les concentrations utilisées, du Diltiazem (DIL) et du Favipiravir (FAV) en combinaison contre un virus recombinant H1N1nLuc.
La figure 3 représente les effets des traitements au Diltiazem et au Baloxavir, ainsi que de la combinaison des deux composés, sur la réplication du virus A/H1N1 puis du virus A/H3N2 en modèle d'épithélium respiratoire humain.
Figure 3a : chronogramme de l'expérience.
Figure 3b : Infection virale par A/H1N1. Comparaison au cours du temps post-infection (pi) du titrage viral sur des épithéliums non traités / traités avec le solvant DMSO / traités avec Diltiazem (90µM) / traités avec Baloxavir (10 nM) / traités avec la combinaison Diltiazem (90µM) et Baloxavir (10 nM)
Figure 3c : Infection virale par A/H1N1. Suivi des mesures de résistance trans-épithéliale (TEER) au cours du temps post-infection (pi), sur les épithéliums tels que décrits ci-dessus dans la légende de Fig. 3b.
Figure 3d : Infection virale par A/H3N2. Comparaison au cours du temps post-infection (pi) du titrage viral sur des épithéliums non traités / traités avec le solvent DMSO / traités avec Diltiazem (90µM) / traités avec Baloxavir (10 nM) / traités avec la combinaison Diltiazem (90µM) et Baloxavir (10 nM)
Figure 3e : Infection virale par A/H3N2. Suivi des mesures de résistance trans-épithéliale (TEER) au cours du temps post-infection (pi), sur les épithéliums tels que décrits ci-dessus dans la légende de Fig. 3d.
La figure 4 représente les effets des traitements de monothérapie au Diltiazem et au Baloxavir sur la réplication du virus A/H1N1 I38T résistant au Baloxavir (Figures 4d, 4e) en comparaison de leurs effets respectifs sur le virus A/H1N1 WT (Figures 4b, 4c) en modèle d'épithélium respiratoire humain.
Figure 4a : Chronogramme de l'expérience.
Figure 4b : Infection virale par A/H1N1. Comparaison au cours du temps post-infection (pi) du titrage viral sur des épithéliums non traités / traités avec le solvant DMSO / traités avec Diltiazem (90µM) / traités avec Baloxavir (10 nM).
Figure 4c : Infection virale par A/H1N1. Suivi des mesures de résistance trans-épithéliale (TEER) au cours du temps post-infection, sur les épithéliums tels que décrits ci-dessus dans la légende de Fig. 4b.
Figure 4d : Infection virale par A/H1N1 I38T (souche résistante au Baloxavir). Comparaison au cours du temps post-infection (pi) du titrage viral sur des épithéliums non traités / traités avec le solvant DMSO / traités avec Diltiazem (90µM) / traités avec Baloxavir (10 nM) / traités avec Baloxavir (100 nM).
Figure 4e : Infection virale par A/H1N1 I38T (souche résistante au Baloxavir). Suivi des mesures de résistance trans-épithéliale (TEER) au cours du temps post-infection, sur les épithéliums tels que décrits ci-dessus dans la légende de Fig. 4d.
La figure 5 représente les effets des traitements au Diltiazem et au Baloxavir, ainsi que de la combinaison des deux composés, sur la réplication de la souche virale résistante au Baloxavir A/H1N1 I38T, sur modèle d'épithélium respiratoire humain.
Figure 5a : Chronogramme de l'expérience.
Figure 5b : Infection virale par /H1N1 I38T (souche résistante au Baloxavir). Comparaison au cours du temps post-infection (pi) du titrage viral sur des épithéliums non traités / traités avec le solvant DMSO / traités avec Diltiazem (90µM) / traités avec Baloxavir (10 nM) / traités avec la combinaison Diltiazem (90µM) et Baloxavir (10 nM).
Figure 5c : Infection virale par /H1N1 I38T. Suivi des mesures de résistance trans-épithéliale (TEER) au cours du temps post-infection, sur les épithéliums tels que décrits ci-dessus dans la légende de Fig. 5b.
Figure 5d : Infection virale par A/H1N1 I38T (souche résistante au Baloxavir). Comparaison au cours du temps post-infection (pi) du titrage viral sur des épithéliums non traités / traités avec Diltiazem (90 µM) / traités avec Baloxavir (100 nM) / traités avec la combinaison Diltiazem (90 µM) et Baloxavir (100 nM).
Figure 5e : Infection virale par A/H1N1 I38T. Suivi des mesures de résistance trans-épithéliale (TEER) au cours du temps post-infection, sur les épithéliums tels que décrits ci-dessus dans la légende de Fig. 5d.
La figure 6 illustre les expériences menées pour observer l'apparition de souches virales résistantes au Baloxavir dans un test classique de passages cellulaires successifs de virus sous pression de sélection antivirale.
Figure 6a : Chronogramme de l'expérience.
Figure 6b : Le graphique représente les concentrations utilisées de Baloxavir et de Diltiazem dans les différents bras expérimentaux (non traité, traitement au Baloxavir, traitement au Diltiazem et traitement en combinaison Diltiazem + Baloxavir) pour chaque passage cellulaire, selon le protocole expérimental décrit précédemment en Fig 6a.

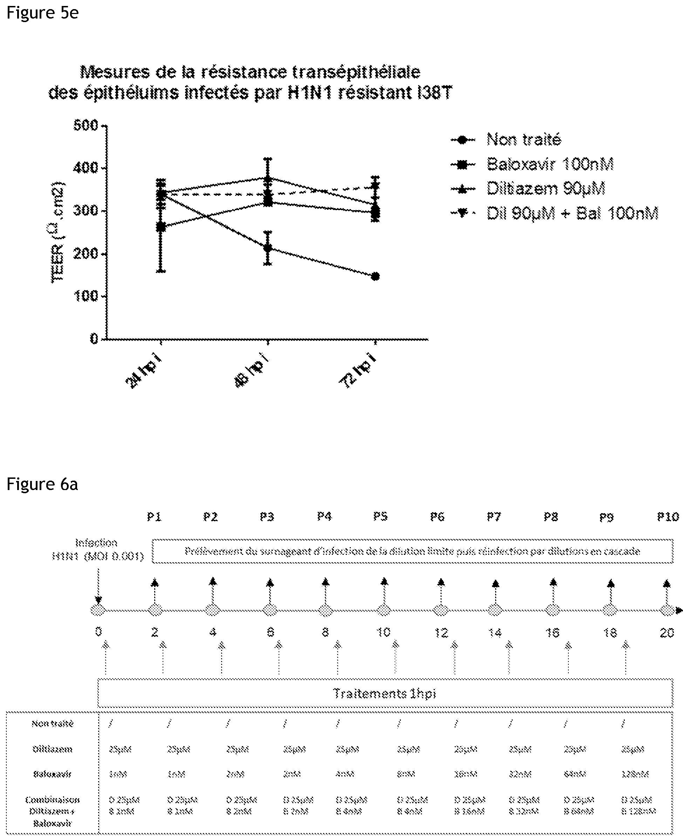

Le tableau présente les dilutions limites des surnageants infectieux issus de chaque passage cellulaire (pour lesquelles il a été observé des effets cytopathiques significatifs) et qui ont été utilisées pour infecter les cellules MDCK de chaque passage cellulaire suivant dans chacun des quatre bras expérimentaux (non traité, traitement au Baloxavir, traitement au Diltiazem et traitement en combinaison Diltiazem + Baloxavir) décrit précédemment en Fig 6a.

Les cinq figures suivantes illustrent la détermination des concentrations médianes inhibitrices (IC50) du Baloxavir sur les virus A/H1N1 issus du dernier passage cellulaire de chacun des bras expérimentaux (non traité, traitement au Baloxavir, traitement au Diltiazem et traitement en combinaison Diltiazem + Baloxavir) décrit précédemment en Fig 6a.

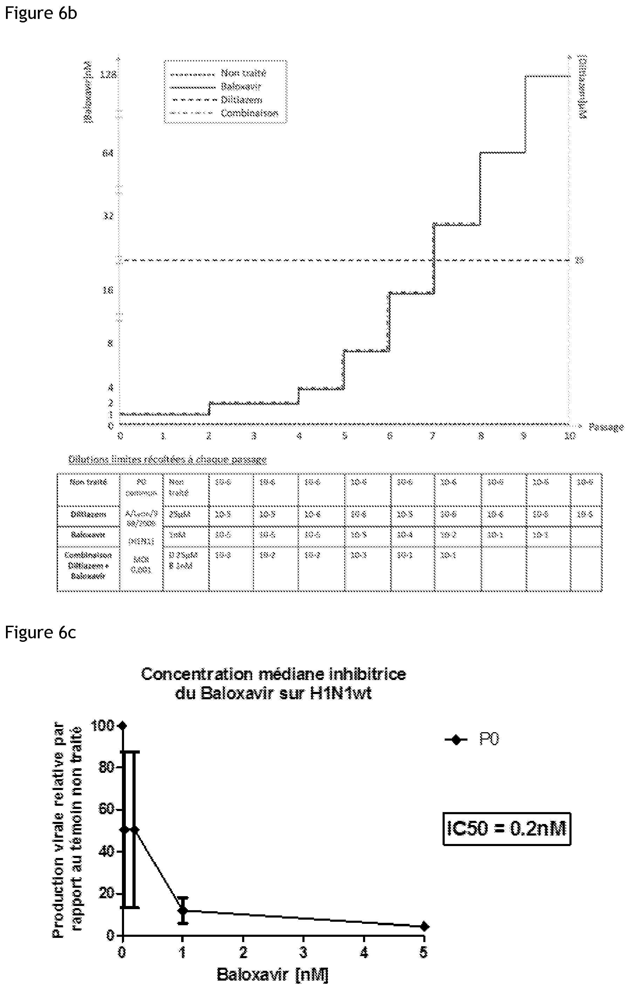

Figure 6c : Détermination de la concentration médiane inhibitrice du Baloxavir sur le virus A/H1N1 issu du passage cellulaire initial P0, en cellules MDCK traitées par différentes concentrations croissantes de Baloxavir. L'IC50 déterminée initialement sur le virus A/H1N1 utilisé pour l'expérimentation, est confirmée à 0.2 nM.

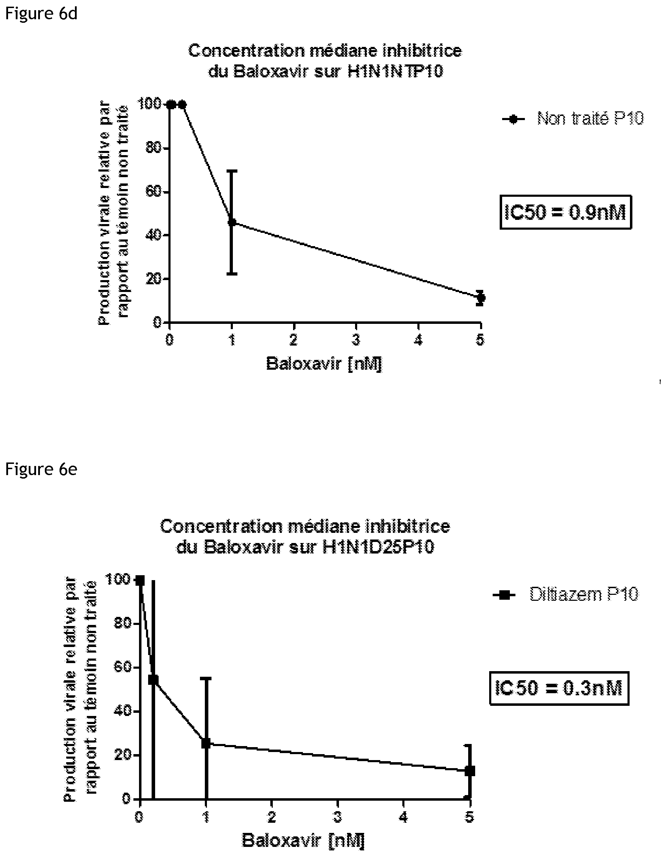

Figure 6d : Détermination de la concentration médiane inhibitrice du Baloxavir sur le virus issu du passage cellulaire P10 du bras expérimental non traité, en cellule MDCK traitées par différentes concentrations croissantes de Baloxavir. L'IC50 calculée du Baloxavir sur le virus A/H1N1 issu du 10ème passage cellulaire sans traitement (non traité), est à 0.9 nM. Cette légère augmentation peut être attribuée à une réplication virale du virus A/H1N1 qui s'est adaptée à l'amplification cellulaire successive sur cellules MDCK dans ce bras expérimental.

Figure 6e : Détermination de la concentration médiane inhibitrice du Baloxavir sur le virus issu du passage cellulaire P10 du bras expérimental de traitement au Diltiazem, en cellule MDCK traitées par différentes concentrations croissantes de Baloxavir. L'IC50 calculée du Baloxavir sur le virus A/H1N1 issu du 10ème passage cellulaire avec traitement au Diltiazem, est à 0.3 nM. Cette IC50 est sensiblement similaire à celle déterminée initialement sur le virus A/H1N1 utilisé pour l'expérimentation.

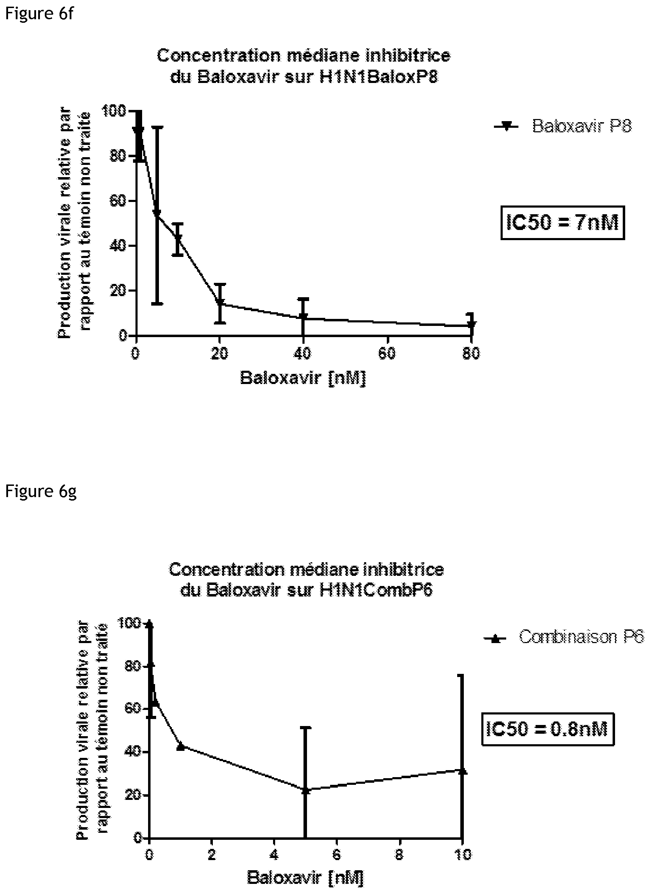

Figure 6f : Détermination de la concentration médiane inhibitrice du Baloxavir sur le virus issu du passage cellulaire P8 du bras expérimental de traitement au Baloxavir, en cellule MDCK traitées par différentes concentrations croissantes de Baloxavir. L'IC50 calculée du Baloxavir sur le virus A/H1N1 issu du 8ème passage cellulaire avec traitement au Baloxavir (en concentration croissante), est à 7 nM. Cette IC50 est très supérieure à celle déterminée initialement sur le virus A/H1N1 utilisé pour l'expérimentation (IC50=0,2nM). Cette augmentation, correspondant à 35 fois l'IC50 initiale, confirme l'émergence d'un virus au phénotype de résistance au Baloxavir.

Figure 6g : Détermination de la concentration médiane inhibitrice du Baloxavir sur le virus issu du passage cellulaire P6 du bras expérimental de traitement par combinaison Diltiazem + Baloxavir, en cellule MDCK traitées par différentes concentrations croissantes de Baloxavir. L'IC50 calculée du Baloxavir sur le virus A/H1N1 issu du 6ème passage cellulaire avec traitement par combinaison au Diltiazem + Baloxavir (en concentration croissante), est à 0,8 nM. Cette IC50 est très similaire à celle calculée pour le Baloxavir sur le virus A/H1N1 issu du 10ème passage cellulaire dans le bras expérimental sans traitement (0.9 nM, cf Fig 6d) et légèrement supérieure à celle calculée initialement sur le virus A/H1N1 utilisé pour l'expérimentation et à l'issue du passage cellulaire P0 (IC50=0,2 nM, cf Fig 6c).

### DESCRIPTION DETAILLEE DE L'INVENTION

La présente invention concerne une combinaison de Diltiazem et de Baloxavir marboxil. L'étendue de l'invention est définie par les revendications.

Par « combinaison », on entend au sens de l'invention une composition comprenant au moins deux composés actifs distincts, les deux composés ayant une action antivirale.

Cette combinaison comprend soit la même quantité en poids de chaque composé antiviral, c'est-à-dire une combinaison de 50% de Diltiazem et 50% de Baloxavir marboxil en poids, soit des doses non égales de chaque composé, telles que 90 % de Diltiazem et 10 % de Baloxavir marboxil, 80 % de Diltiazem et 20 % de de Baloxavir marboxil, 70 % de Diltiazem et 30 % de Baloxavir marboxil, 60 % de Diltiazem et 40 % de Baloxavir marboxil, 40 % de Diltiazem et 60 % de Baloxavir marboxil, 30 % de Diltiazem et 70 % de Baloxavir marboxil, 20 % de Diltiazem et 80 % de Baloxavir marboxil, ou bien encore 10 % de Diltiazem et 90 % de Baloxavir marboxil, les pourcentages étant exprimés en poids du composé par rapport au poids total de la combinaison.

Selon l'invention, la combinaison comprend du Diltiazem et du Baloxavir marboxil. Plus particulièrement, la combinaison consiste en du Diltiazem et du Baloxavir marboxil. Dans une variante de l'invention, la combinaison comprend du Diltiazem et du Baloxavir, sous sa forme active (acide de Baloxavir).

Est également ici décrite une combinaison comprenant du Diltiazem et du Pimodivir.

Est également ici décrite une combinaison comprenant du Diltiazem et du RO-7.

Est également ici décrite une combinaison comprenant du Diltiazem et du CC-42344.

Selon d'autres mises en œuvre, la combinaison comprend :
- du Diltiazem, du Baloxavir marboxil et du CC-42344 ;
- du Diltiazem, du Baloxavir marboxil et du Pimodivir;
- du Diltiazem, du Baloxavir marboxil et du RO-7.

Ainsi, la combinaison selon l'invention comprend ou consiste en au moins deux composés actifs à activité antivirale, qui sont décrits en détails ci-dessous.

Cette combinaison présentant des effets antiviraux synergiques, l'un de ses avantages est que les doses utilisées de chaque composé antiviral peuvent être diminuées par rapport aux doses classiquement utilisées en monothérapie. Ceci permet d'une part de limiter l'apparition d'effets secondaires négatifs sur les organismes traités, et d'autre part de limiter l'apparition de résistance chez les virus auxdits composés antiviraux.

Cette demande de brevet décrit également une combinaison de Diltiazem et d'au moins un composé inhibiteur de polymérase(s) virale(s) tel que décrit ci-après.

### Le Diltiazem

Le Diltiazem est une molécule membre de la famille des benzothiazépines, référencée sous le numéro CAS 42399-41-7. Cette molécule peut être sous la forme de deux énantiomères L-cis et D-cis, ou d'un mélange racémique.

La formule chimique développée de l'hydrochloride de Diltiazem est représentée ci-dessous, en formule (I) :

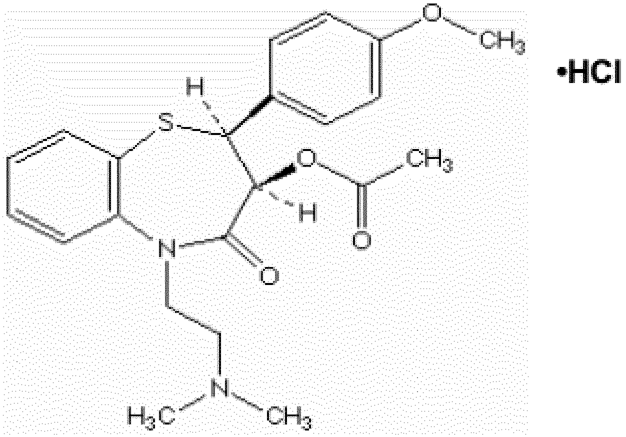

Le Diltiazem est connu depuis plus de 30 ans et est approuvé, en Europe et aux Etats-Unis, par les autorités règlementaires du médicament. Il peut être administré sous forme d'hydrochloride de Diltiazem. Cardizem^{®}, Cartia^{®}, Taztia^{®} et Dilacor^{®} sont ses dénominations commerciales les plus courantes.

De nombreuses formulations sont disponibles, en particulier des formulations à libération prolongée. Le Diltiazem est disponible sous différentes formes galéniques, telles que sous forme de crème pour application topique, sous forme de comprimés ou capsules pour administration orale, sous forme de poudre pour préparation de solution injectable ou sous forme de préparations pharmaceutiques pour inhalation (WO 02/094238, US 4,605,552).

La posologie classique chez l'Homme est de 180 à 360 mg/jours, administrés en capsule ou comprimés, pour son utilisation thérapeutique en tant qu'inhibiteur des canaux calciques.

La propriété physiologique identifiée en premier lieu de ce composé est l'inhibition des canaux calciques, et donc l'inhibition des flux de calcium intracellulaires. Le Diltiazem freine notamment l'entrée du calcium transmembranaire au niveau de la fibre musculaire myocardique et de la fibre musculaire lisse des vaisseaux. Ceci permet de diminuer la concentration intracellulaire calcique atteignant les protéines contractiles.

Chez l'homme, l'administration de Diltiazem est indiquée pour son action vasodilatatrice, dans le but de réduire le travail cardiaque. Il est ainsi utilisé dans la prise en charge des désordres cardiaques et circulatoires tels que les angines de poitrine, l'hypertension artérielle, les ischémies myocardiques et la tachycardie.

Le Diltiazem agit également en renversant les effets de l'angiotensine II, du point de vue rénal et périphérique.

En application topique, le Diltiazem peut être indiqué en cas de fissures anales chroniques.

Le brevet EP 1 117 408 décrit l'utilisation de Diltiazem, en tant que composé inhibiteur des canaux calciques, pour traiter des pathologies liées à la dégénérescence des photorécepteurs de la rétine.

En ce qui concerne l'utilisation du Diltiazem pour le traitement des infections virales, comme précédemment évoqué, celle-ci a déjà été décrite dans plusieurs demandes de brevet. De plus, un essai clinique est en cours à ce jour (FLUNEXT PHRC #15-0442 ClinicalTrials.gov Identifier: NCT03212716), dans le but d'obtenir l'autorisation de mise sur le marché pour cette nouvelle indication thérapeutique antivirale.

### Les inhibiteurs du complexe polymérase viral

Le complexe polymérase des virus influenza est composé de 3 sous-unités PB1 (Protéine Basique 1), PB2 (Protéine Basique 2) et PA (Protéine Acide). Ce complexe permet la transcription et la réplication du génome viral.

Des traitements antiviraux en cours de développement et/ou ayant eu une autorisation récente de mise sur le marché ciblent les différentes sous-unités de ce complexe polymérase et agissent selon différents mécanismes d'actions :
- le Baloxavir marboxil est un inhibiteur de l'endonucléase médiée par la sous-unité PA,
- le Pimodivir est un inhibiteur de la sous-unité PB2, et
- le Favipiravir est un analogue nucléosidique, inhibiteur de l'élongation de l'ARN par le complexe polymérase.

Le premier composé inhibiteur de ce complexe polymérase à être autorisé par les autorités règlementaires, et donc commercialisé, a été le Baloxavir marboxil. Aujourd'hui, d'autres inhibiteurs de PB2 (Pimodivir) et PB1 (Favipiravir) sont respectivement testés en essais cliniques de phase 3, ou possèdent une approbation restreinte.

### Baloxavir marboxil

Le Baloxavir marboxil (S-033188), également désigné Baloxavir dans la présente demande (notamment dans les figures), est une molécule inhibant l'activité endonucléase dépendante de la coiffe de la sous-unité PA du complexe polymérase des virus influenza. Cette molécule est une pro-drogue qui, après administration au sein d'un organisme vivant, est métabolisée en sa forme active « acide de Baloxavir » qui inhibe l'initiation de la synthèse de l'ARNm viral en formant des liaisons stables avec deux ions manganèse dans le site actif de la sous-unité PA du complexe polymérase virale. La métabolisation de la pro-drogue en métabolite actif est rapide mais l'élimination hépatique du Baloxavir acide est longue (demi-vie de 50 à 90 heures) et permet l'administration par voie orale d'une dose unique (Hayden & Shindo, 2019). Dans la présente demande, le terme « Baloxavir » désigne la forme active « acide de baloxavir » de numéro CAS 1985605-59-1, obtenue après métabolisation de la pro-drogue Baloxavir marboxil de numéro CAS 1985606-14-1.

Cette forme active est utilisée pour toutes les expérimentations *in vitro.* Les premières études *in vitro* ont montré une très bonne efficacité pour inhiber les virus influenza A (CE50 moyenne de 1,4 à 3,1 nM) et B (CE50 moyenne de 4,5 à 8,9 nM). Le Baloxavir s'est également montré efficace sur des virus résistants aux adamantanes et aux inhibiteurs de neuraminidase (H274Y) et sur différents sous-types de virus influenza A issus du réservoir aviaire. L'association de Baloxavir et d'un inhibiteur de neuraminidase (Oseltamivir) a montré un effet synergique permettant de protéger des souris d'une infection létale par le virus influenza A(H1N1). Cette association permet également une baisse significative du titre viral dans les poumons, 24 heures après la mise en route du traitement (Fukao et al., 2019).

Le Baloxavir marboxil a le potentiel de révolutionner le traitement des infections grippales étant donné sa plus longue demi-vie permettant un régime thérapeutique comportant une seule dose (contrairement à 5 jours de traitement pour l'Oseltamivir) et une activité antivirale majorée par rapport aux inhibiteurs de la Neuraminidase.

Dans une étude clinique récente de phase 3, le temps médian pour la cessation de l'excrétion virale chez les adultes infectés par influenza non hospitalisés était de 48 h pour le groupe traité au Baloxavir marboxil, comparé à 72 h pour le groupe traité à l'Oseltamivir, et 96 h pour le groupe placebo, bien que la différence dans la durée des symptômes fût non significative entre les 2 types de traitement (Hayden *et al.,* 2018).

Le Baloxavir marboxil est très bien toléré avec des effets secondaires principalement digestifs (nausée, diarrhée...) chez 4 à 5 % des patients auxquels ce composé a été administré.

En revanche, un des inconvénients majeurs de cette molécule est la rapide apparition de mutations de résistances au sein de la sous-unité PA du complexe polymérase des virus influenza. Dès les premiers tests *in vitro* des virus variants ont été isolés avec une substitution principale sur l'acide aminé I38 (I38T/M/ ou phénylalanine F) de PA entrainant une diminution de sensibilité des virus au Baloxavir de 10 à 100 fois (Noshi *et al.,* 2018). En clinique, bien que très rares à ce jour, ces substitutions peuvent être retrouvées en l'absence de traitement antiviral (Takashita *et al.,* 2019). Lors des essais cliniques, les substitutions I38T/M dans PA étaient retrouvées dans 2 à 24 % des patients traités par Baloxavir marboxil. De plus, la durée des signes cliniques était moins réduite chez les patients traités par Baloxavir marboxil et présentant un virus muté.

Récemment, des effets antiviraux du Baloxavir marboxil ont été observés sur d'autres types de virus à génome segmenté d'ARN de polarité négative, tel que le Severe Fever with Thrombocytopenia Syndrome Virus (SFTSV) et le Heartland Virus (HRTV), pathogènes issus des tiques. (Wang *et al.,* 2020)

### Pimodivir

Le Pimodivir (JNJ63623872 ou JNJ-872 ou VX-787) est un composé qui inhibe la sous-unité PB2 du complexe polymérase des virus influenza A en se liant au site de liaison de la coiffe, empêchant la liaison du ligand naturel 7-methyl GTP et la synthèse d'ARNm viral (Clark et al., 2014).

Le Pimodivir est bien toléré avec des effets secondaires principalement digestifs (nausée, diarrhée...) dépendant de la dose.

Deux essais cliniques de phase III ont été menés lors de la saison 2017-2018.

Comme pour tout traitement anti-infectieux, des mutations de résistances dans PB2 ont été identifiées in vitro et in vivo. La substitution M431I dans PB2 serait responsable d'une baisse de la sensibilité d'environ 60 fois et a été retrouvée chez 10 % des patients traités en monothérapie avec du Pimodivir (Trevejo *et al.,* 2018).

### RO-7

Le RO-7 est un inhibiteur de l'activité endonucléase de la protéine acide PA du complexe polymérase des virus influenza, découvert en 2016. Ce composé a une bonne activité in vitro (CE50 de 1,1 à 21,6 nM) sur une large sélection de virus influenza circulant chez l'Homme ou présents dans le réservoir aviaire. L'utilisation du RO-7 a protégé des souris d'une infection létale par les virus influenza tout en diminuant significativement la charge virale dans leurs poumons. Comme le RO-7 est structuralement très proche du Baloxavir marboxil, les substitutions de l'acide aminé I38 de la PA entrainent également une diminution de la sensibilité des virus influenza à cette molécule.

### CC-42344

Le CC-42344 est un des composés les plus récemment identifiés. Il agit en inhibant l'activité de la sous-unité PB2 du complexe polymérase des virus influenza. Les données précliniques ne sont pas encore publiées mais cette molécule présenterait une bonne activité antivirale et pourrait être administrée par voie orale, en IV ou en inhalation.

### Ribavirine

La ribavirine (Virazole) est une molécule antivirale à large spectre qui est active contre de nombreux virus à ADN et à ARN. C'est un analogue de la guanosine qui, dans sa forme monophosphatée, inhibe l'IMPDH (inosine 5'-monophosphate dehydrogenase) et diminue les taux de GTP disponibles. Sous forme triphosphatée, elle s'incorpore dans l'ARN viral transcrit, et ainsi bloque l'élongation de l'ARN et a un effet mutagène. Elle est donc considérée comme un inhibiteur de la réplication du génome viral.

Dans les études in vitro, la ribavirine s'était avérée efficace contre tous types de virus influenza. En revanche, les études cliniques ont conclu que la ribavirine administrée par voie orale n'était pas efficace contre les virus de la grippe. De plus les nombreux effets secondaires observés ont limité son utilisation. Toutefois, plusieurs tests cliniques basés sur une association de la ribavirine avec d'autres composés antiviraux sont en cours chez l'Homme.

### Favipiravir

Le Favipiravir ou T-705 est un composé utilisé comme antiviral contre les virus à ARN, notamment les orthomyxovirus, incluant notamment les différents virus influenza, le virus du Nil occidental, le virus de la fièvre jaune, le virus de la fièvre aphteuse, ainsi que d'autres flavivirus, arénavirus, bunyavirus et alphavirus.

Cette molécule agirait par inhibition sélective de la polymérase de ces virus (Yousuke et al., 2009).

En ce qui concerne son effet sur les virus influenza, le Favipiravir a montré une efficacité en culture cellulaire MDCK, contre des virus influenza A, B et C, y compris des virus résistants aux inhibiteurs de neuraminidase, ainsi qu'une efficacité en modèle murin infecté par la souche A/PuertoRico/8/34. Le Favipiravir agirait comme un analogue de nucléoside présentant une faible cytotoxicité et capable d'inhiber spécifiquement la polymérase des virus influenza.

In vivo, le Favipiravir s'est avéré efficace pour protéger des souris infectées par du virus A/H5N1 hautement pathogène, sensible ou résistant à l'Oseltamivir.

L'association d'Oseltamivir et de Favipiravir s'est avérée synergique à certaines concentrations chez des souris infectées par des virus influenza de type A.

Des mutations de résistance au Favipiravir ont été identifiées dans la polymérase des virus influenza (Goldhill et al., 2018).

L'utilisation thérapeutique chez l'Homme de ce composé est autorisée par une AMM au Japon depuis 2014.

### Utilisation thérapeutique de ladite combinaison

La présente description décrit une combinaison de Diltiazem et d'au moins un composé inhibiteur de polymérase(s) virale(s) choisi parmi le Baloxavir marboxil, le Pimodivir, le RO-7 et le CC-42344, pour son utilisation en tant que médicament.

La présente description divulgue notamment une combinaison de Diltiazem et d'au moins un composé inhibiteur de polymérase virale choisi parmi le Baloxavir marboxil, le Pimodivir, le RO-7 et le CC-42344, pour son utilisation thérapeutique dans la prévention et/ou le traitement d'une infection virale, notamment des voies respiratoires et/ou intestinales d'un organisme humain ou animal.

Le terme « prévention » désigne le fait d'empêcher, ou du moins de diminuer la probabilité de, l'apparition d'une infection virale dans un organisme humain ou animal.

Le terme « traitement » désigne le fait de combattre l'infection virale dans un organisme humain ou animal. Cela signifie administrer un traitement dans le but de diminuer la charge virale au sein de l'organisme. Le terme « traitement » désigne aussi le fait d'atténuer les symptômes associés à l'infection virale (fièvre, fatigue).

Ledit traitement est applicable aussi bien à l'être humain qu'aux animaux, et en particulier aux animaux d'élevage comme les porcs, les chevaux et les volailles.

La présente description divulgue une combinaison de Diltiazem et d'au moins un composé inhibiteur de polymérase virale choisi parmi le Baloxavir marboxil, le Pimodivir, le RO-7 et le CC-42344, pour son utilisation thérapeutique dans la prévention d'une infection virale.

La présente description divulgue aussi une combinaison de Diltiazem et d'au moins un composé inhibiteur de polymérase virale choisi parmi le Baloxavir marboxil, le Pimodivir, le RO-7 et le CC-42344, pour son utilisation thérapeutique dans le traitement d'une infection virale.

La présente description divulgue aussi une combinaison de Diltiazem et d'au moins un composé inhibiteur de polymérase virale choisi parmi le Baloxavir marboxil, le Pimodivir, le RO-7 et le CC-42344, pour son utilisation thérapeutique dans la prévention et le traitement d'une infection virale.

Ladite combinaison comprend en particulier du Diltiazem et du Baloxavir marboxil.

La présente description divulgue également une méthode pour traiter un patient atteint d'une infection virale, notamment des voies respiratoires et/ou intestinales, comprenant l'administration audit patient d'une combinaison de Diltiazem et d'au moins un composé inhibiteur de polymérase(s) virale(s) choisi dans le groupe constitué de : Baloxavir marboxil, Pimodivir, RO-7 et CC-42344.

La présente description divulgue aussi une méthode pour prévenir l'apparition d'une infection virale, notamment des voies respiratoires et/ou intestinales, chez un individu susceptible d'être infecté par un virus, comprenant l'administration audit individu d'une combinaison de Diltiazem et d'au moins un composé inhibiteur de polymérase(s) virale(s) choisi dans le groupe constitué de : le Baloxavir marboxil, le Pimodivir, le RO-7 et le CC-42344.

La présente description divulgue également une méthode vétérinaire pour prévenir et/ou traiter une infection virale, notamment des voies respiratoires et/ou intestinales, chez un animal infecté ou susceptible de l'être, comprenant l'administration audit animal d'une combinaison de Diltiazem et d'au moins un composé inhibiteur de polymérase(s) virale(s) choisi dans le groupe constitué de : le Baloxavir marboxil, le Pimodivir, le RO-7 et le CC-42344.

### Les infections virales et virus associés

On entend au sens de l'invention par « infection virale » une maladie provoquée par un virus ayant pénétré dans l'organisme, en infectant certaines cellules dudit organisme désignées par les appellations « cellules cibles » ou « cellules hôtes ». Une infection virale est généralement diagnostiquée par un professionnel de santé, sur la base de l'observation des symptômes du patient ou de l'animal infecté. Des examens biologiques complémentaires peuvent être nécessaires pour confirmer le diagnostic (analyses de sang et/ou des expectorations et/ou du liquide bronchoalvéolaire et / ou d'échantillons biologiques issus du tractus intestinal).

La combinaison ici décrite est destinée à prévenir et/ou traiter une infection virale, notamment des voies respiratoires et/ou intestinales d'un organisme humain ou animal, autrement dit une infection touchant les cellules des épithéliums respiratoires.

Par « infection des voies respiratoires », on entend les infections virales touchant les poumons et les voies respiratoires c'est-à-dire les voies de passage de l'air pour respirer. Elles comprennent notamment le rhume, la grippe et la bronchiolite.

Les virus impliqués dans les infections respiratoires virales incluent en particulier le virus respiratoire syncytial (VRS), les virus influenza (la grippe), les virus parainfluenza, les adénovirus et les rhinovirus.

Chez l'enfant, les principales causes d'infections virales respiratoires sont les rhinovirus, les virus influenza, les virus parainfluenzae, le virus respiratoire syncytial (VRS), les entérovirus, les coronavirus et certaines souches d'adénovirus.

Dans le cadre de la présente description, il s'agira en particulier d'une infection respiratoire aigüe (IRA).

Par « infection des voies intestinales », on entend les infections virales touchant l'appareil digestif qui comprend notamment, de haut en bas : la bouche, le pharynx (au croisement des voies digestives et respiratoires), l'œsophage, l'estomac, et les intestins.

Les causes les plus fréquentes de gastro-entérite sont liées à des infections virales par des norovirus ou rotavirus.

La combinaison thérapeutique selon l'invention est destinée au traitement et/ou à la prévention d'une infection par un virus influenza.

Les virus influenza, responsables de la grippe, sont divisés en quatre types : A, B, C et D. A la surface du virus se trouvent deux glycoprotéines qui jouent un rôle important dans l'infection des cellules de l'organisme infecté : l'hémagglutinine (HA) et la neuraminidase (NA). Il existe différents sous-types de virus influenza A selon la nature des glycoprotéines HA et NA à leur surface : 16 types d'HA et 9 types de NA ont été identifiés chez les virus circulants dans le monde animal et notamment parmi les oiseaux migrateurs marins. On peut ainsi définir les virus influenza selon le type de glycoprotéines qu'ils possèdent à leur surface.

Chez l'homme, les virus de sous-type A circulants depuis plusieurs décennies sont les sous-types H1N1, H2N2 et H3N2, avec des transmissions inter-espèces occasionnelles, notamment de l'animal à l'homme, des virus aviaires H5N1, H7N7, H7N9, H5N2 et H9N2. Comme le souligne la récente émergence d'un nouveau virus grippal pandémique H1N1 d'origine porcine, aviaire et humaine (virus avec réassortiment porcin, aviaire et humain), les virus influenza de type A représentent une menace sérieuse en santé publique. Les pandémies de grippe sont le résultat notamment de cassures antigéniques qui correspondent à l'apparition de virus dotés de nouvelles glycoprotéines de surface (HA et NA) dans la population humaine. Ces cassures permettent la transmission directe chez l'homme de virus animaux et notamment aviaires : c'est le cas des épidémies de H5N1 aviaires hautement pathogènes depuis 2003 en Asie, ou de des épidémies de grippe H7N7 aux Pays-Bas en 2003 et H7N9 dans le sud-est asiatique en 2013. Par ailleurs, les épidémies de grippe saisonnières, qui sont notamment le résultat de dérive génétique (apparition de mutations dans les glycoprotéines de surface) sont une cause majeure de morbidité et de mortalité accrue dans la population humaine, surtout chez les individus très jeunes, les personnes âgées, les individus immunodéprimés et les porteurs de maladies cardio-pulmonaires.

On entend au sens de l'invention par « virus influenza » les agents étiologiques de la grippe de type A, B, C ou D, et notamment les virus influenza de type A et de type B, ayant pour hôte l'homme ou l'animal. Les termes « virus de la grippe » et « virus influenza » sont utilisés indifféremment dans la demande et désignent les mêmes virus.

L'invention concerne une combinaison de Diltiazem et de Baloxavir marboxil pour son utilisation thérapeutique dans la prévention et/ou le traitement d'une infection par un virus influenza. Dans une variante de l'invention, celle-ci concerne une combinaison de Diltiazem et d'acide de Baloxavir pour son utilisation thérapeutique dans la prévention et/ou le traitement d'une infection par un virus influenza.

Selon un aspect de l'invention, les combinaisons de la présente invention sont utilisées pour la prévention et/ou le traitement des infections par au moins un virus influenza de type A.

Avantageusement, les combinaisons de la présente invention ont un spectre d'action large contre les différents sous-types des virus influenza de type A.

Selon un aspect particulier, le virus de la grippe est un virus de type A choisi parmi les sous-types H1N1, H2N2, H3N2, H5N1, H7N7, H7N9, H5N2 et H9N2.

Selon un autre aspect de l'invention, les combinaisons de la présente invention sont utilisées pour la prévention et/ou le traitement des infections par au moins un virus influenza de type B.

### Résistance des virus

L'invention concerne en particulier une combinaison de Diltiazem et de Baloxavir marboxil, pour son utilisation thérapeutique dans la prévention et/ou le traitement d'une infection par un virus influenza, ledit virus influenza étant résistant à l'action inhibitrice d'au moins un composé antiviral, notamment d'un composé anti-grippal, et plus particulièrement d'un inhibiteur de polymérase(s) virale(s).

En effet, la combinaison selon l'invention est particulièrement intéressante dans le cas où le virus causant l'infection virale n'est pas ou peu sensible à l'action d'un composé inhibiteur de polymérase(s) virale(s) classiquement utilisé, et en particulier choisi parmi le Baloxavir marboxil, le Pimodivir, le RO-7 et le CC-42344.

Comme présenté ci-dessus, l'inconvénient majeur de l'utilisation thérapeutique de cette famille de composés qui ciblent les polymérases virales est le développement de souches virales résistantes à leur action, devenant insensibles à l'action inhibitrice de ces composés.

Les avantages de l'utilisation combinée de Diltiazem et de Baloxavir marboxil sont les suivants :
1) l'émergence de souches virales résistantes à au moins un composé inhibiteur de polymérase virale administré est nettement limitée, voire totalement inhibée, lors d'une administration combinée avec du Diltiazem (voir exemple 6) ;
2) le Diltiazem seul a une activité antivirale effective contre des souches virales résistantes à un inhibiteur de polymérase virale (voir exemple 4, basé sur une souche virale résistante au Baloxavir) ;
3) la combinaison Diltiazem + Baloxavir marboxil a une activité antivirale effective contre des souches virales résistantes au Baloxavir, du fait de la présence de Diltiazem et d'une forte concentration de Baloxavir (voir exemple 5).

Une combinaison selon l'invention comprend du Diltiazem et du Baloxavir marboxil, et est utilisée pour le traitement et/ou la prévention d'une infection par un virus influenza résistant à l'action inhibitrice d'un inhibiteur de polymérase(s) virale(s), par exemple un virus présentant une mutation de résistance dans la sous-unité PB2 de la polymérase.

Selon une autre mise en œuvre de l'invention, la combinaison comprend du Diltiazem et du Baloxavir marboxil, et est utilisée pour le traitement et/ou la prévention d'une infection par un virus influenza résistant à l'action inhibitrice du Baloxavir marboxil. De manière particulière, il pourra s'agir d'une souche virale influenza A/H1N1 portant une mutation dans la sous-unité PA de la polymérase. Plus précisément, il pourra s'agir d'une souche virale influenza A/H1N1 portant une mutation ponctuelle I38T dans la sous-unité PA de la polymérase.

Selon une autre mise en œuvre de l'invention, le virus influenza est résistant à l'action inhibitrice d'au moins un composé anti-grippal, notamment un composé inhibiteur de neuraminidase tel que l'Oseltamivir (Tamiflu^{®}).

Est également décrit ici le Diltiazem pour son utilisation thérapeutique dans la prévention et/ou le traitement d'une infection par un virus influenza, ledit virus influenza étant résistant à l'action inhibitrice d'au moins un composé antiviral, notamment d'un composé anti-grippal, et plus particulièrement d'un inhibiteur de polymérase(s) virale(s). En particulier, ce virus influenza sera résistant à l'un des composés suivants : Baloxavir marboxil, Pimodivir, RO-7, CC-42344 et un inhibiteur de neuramidase, notamment l'Oseltamivir.

### Autre(s) agent(s) actif(s) présent(s) dans la combinaison

La présente invention concerne également une combinaison de Diltiazem et de Baloxavir marboxil, comprenant de plus un autre agent actif, notamment un agent antiviral et/ou un antibiotique, pour son utilisation thérapeutique dans la prévention et/ou le traitement d'une infection virale par un virus influenza.

Selon un aspect préféré, l'agent antiviral est choisi parmi les agents antiviraux bien connus de l'homme du métier, classiquement utilisés pour prévenir ou traiter la grippe. De tels agents antiviraux actifs sur au moins un virus influenza sont disponibles dans le commerce, et décrits dans des ouvrages de référence comme Le Dictionnaire Vidal. On peut citer notamment l'Oseltamivir. Ainsi, un objet de la présente invention est une combinaison de Diltiazem, Baloxavir marboxil et d'Oseltamivir, pour son utilisation thérapeutique dans la prévention et/ou le traitement d'une infection virale par un virus influenza.

L'antibiotique est choisi parmi les antibiotiques bien connus de l'Homme du métier, notamment ceux utilisés lors des infections virales pour éviter la surinfection bactérienne, et notamment ceux de la famille des macrolides, et tout particulièrement la roxythromycine.

### Composition pharmaceutique ou vétérinaire

La présente invention est également relative à une composition pharmaceutique comprenant, dans un véhicule pharmaceutique adapté, une combinaison de Diltiazem et de Baloxavir marboxil, pour son utilisation thérapeutique dans la prévention et/ou le traitement d'une infection virale par un virus influenza.

Est également partie de l'invention une composition vétérinaire comprenant, dans un véhicule pharmaceutique adapté, une combinaison de Diltiazem et de Baloxavir marboxil, pour son utilisation vétérinaire dans la prévention et/ou le traitement d'une infection virale par un virus influenza.

Cette composition pharmaceutique ou vétérinaire comprend une quantité efficace de Diltiazem et une quantité efficace de Baloxavir marboxil.

Par « quantité efficace », on entend au sens de l'invention une quantité de composé antiviral suffisante pour inhiber la prolifération et/ou la réplication du virus, et/ou le développement de l'infection virale au sein de l'organisme. Cette inhibition peut être quantifiée, par exemple en mesurant la production virale comme cela est présenté dans les exemples de la présente demande.

Par exemple, *in vitro,* les quantités dites « efficaces » sont les suivantes :
- pour le Diltiazem, une concentration de 5 à 200 µM est préférée, et
- pour le Baloxavir (forme active), une concentration de 5 à 200 nM est préférée.

Selon l'invention, le terme « véhicule pharmaceutique » désigne un ou des véhicules ou des excipients pharmaceutiques acceptables selon l'invention, c'est à dire des véhicules ou des excipients dont l'administration à un individu ou un animal ne s'accompagne pas d'effets délétères significatifs, et qui sont bien connus de l'homme du métier.

Les compositions pharmaceutiques ou vétérinaires selon la présente invention sont adaptées pour une administration orale, sublinguale, par inhalation, sous-cutanée, intramusculaire, intraveineuse, transdermique, oculaire ou rectale.

Selon un aspect préféré de l'invention, la composition pharmaceutique est caractérisée en ce qu'elle est sous une forme galénique adaptée pour une administration par inhalation.

L'inhalation désigne l'absorption par les voies respiratoires. C'est en particulier une méthode d'absorption de composés à des fins thérapeutiques, de certaines substances sous forme de gaz, de micro-gouttelettes ou de poudre en suspension.

L'administration de compositions pharmaceutiques ou vétérinaires par inhalation, c'est-à-dire par les voies nasale et/ou buccale, est bien connue de l'Homme du métier.

On distingue deux types d'administration par inhalation :
- l'administration par insufflation lorsque les compositions sont sous la forme de poudres, et
- l'administration par nébulisation lorsque les compositions sont sous la forme d'aérosols (suspensions) ou sous la forme de solutions, par exemple de solutions aqueuses, mises sous pression. L'utilisation d'un nébuliseur ou d'un pulvérisateur sera alors recommandée pour administrer la composition pharmaceutique ou vétérinaire.

La forme galénique considérée ici est donc choisie parmi : une poudre, une suspension aqueuse de gouttelettes ou une solution sous pression.

Est également ici décrite une composition pharmaceutique comprenant, dans un véhicule pharmaceutique adapté, une combinaison de Diltiazem et d'au moins un composé inhibiteur de polymérase(s) virale(s) choisi parmi le Baloxavir marboxil, le Pimodivir, le RO-7 et le CC-42344, pour son utilisation en tant que médicament.

Est également ici décrite une composition pharmaceutique comprenant, dans un véhicule pharmaceutique adapté, une combinaison de Diltiazem et d'au moins un composé inhibiteur de polymérase(s) virale(s) choisi parmi le Baloxavir marboxil, le Pimodivir, le RO-7 et le CC-42344, pour son utilisation thérapeutique dans la prévention et/ou le traitement d'une infection virale, notamment des voies respiratoires et/ou intestinales d'un être humain ou d'un animal, plus particulièrement d'une infection par un virus influenza.

### Produit de combinaison

La présente invention concerne aussi un produit de combinaison comprenant du Diltiazem et du Baloxavir marboxil, pour son utilisation simultanée, séparée ou séquentielle dans la prévention et/ou le traitement d'une infection virale par un virus influenza.

Ce produit de combinaison pourra être utilisé chez l'Homme ou chez l'animal.

Il pourra comprendre d'autres composés actifs, et notamment de l'Oseltamivir.

### EXEMPLES

Dans les exemples suivants, le terme Baloxavir est utilisé pour désigner l'acide de Baloxavir, c'est-à-dire la forme activée du composé, et non la forme administrée à un patient qui est une prodrogue.

### Exemple 1 - La combinaison Diltiazem + Baloxavir a un effet antiviral synergique contre un virus recombinant H1N1nLuc en système cellulaire

Des cellules A549 infectées par A/H1N1nLuc (Multiplicity of infection (MOI) 0,01) ont été traitées une heure post-infection par des concentrations croissantes de Diltiazem (6 nM à 100 µM), d'acide de Baloxavir (0.0625 nM à 1 nM, commercialisé par Med Chem Tronica, ref. HY-109025A) seul et en combinaison.

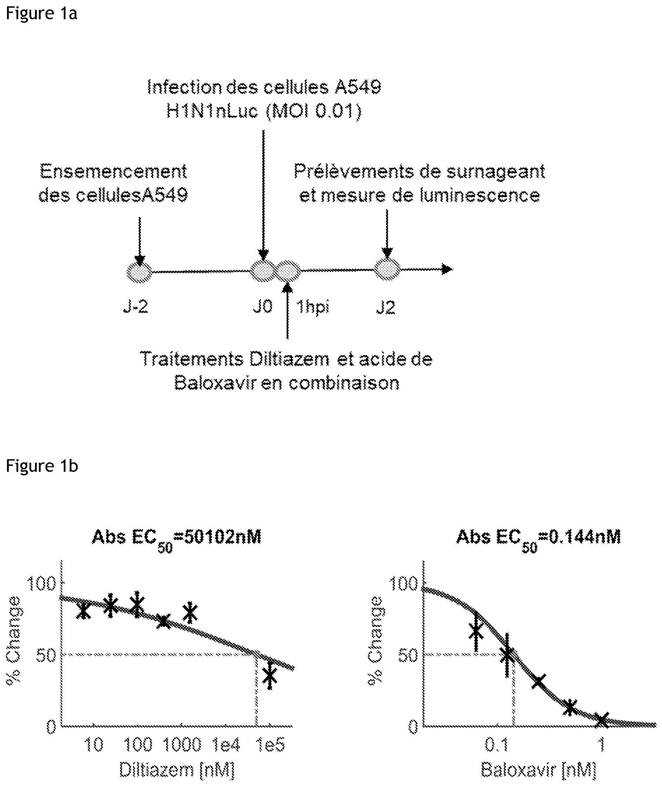

La luminescence mesurée dans les surnageants cellulaires d'infection (prélevés à 48 heures post-infection) reflète le niveau de réplication virale (chronogramme d'expérience représenté en Fig 1a). Les EC50 de chacune des deux molécules ont été déterminés (EC50 Diltiazem= 50102 nM ; EC50 Baloxavir= 0,144 nM, cf Fig 1b).

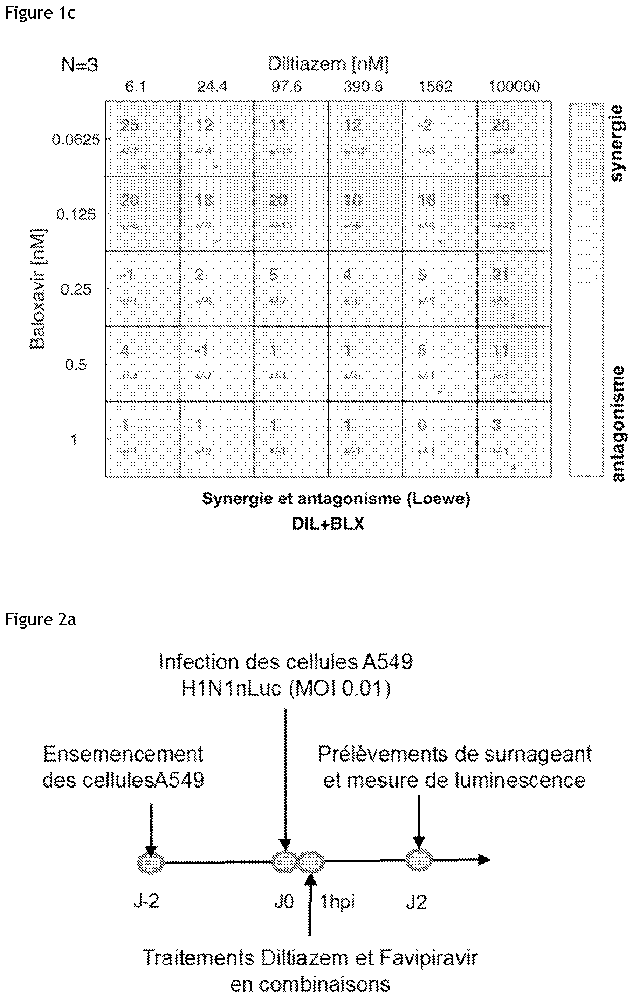

Les effets de la combinaison des molécules ont été caractérisés par l'analyse via le logiciel Combenefit des données de luminescence mesurées (représentation sous forme d'un tableau avec une échelle chromatique, cf Fig 1c).

L'effet synergique ou antagoniste de la combinaison est représenté par le « coefficient de synergie » (Synergy Score, analyse selon modèle de Loewe), avec une échelle chromatique afin de faciliter l'interprétation des résultats. Un coefficient ≥ à 5 (gris à gris foncé) indique un effet synergique de la combinaison de molécules, tandis qu'un coefficient ≤ à -5 (blanc) indique un effet antagoniste de la combinaison de molécules. Des coefficients entre -5 et 5 sont indicatifs d'une « non-interference » des molécules dans la combinaison, voire d'effet additif entre les différents traitements. Nombre d'expériences = 3.

Comme cela est présenté en figure 1c, la combinaison du Diltiazem et d'acide de Baloxavir présente des effets synergiques.

Deux conditions particulières sont particulièrement synergiques :
(i) les conditions associant une forte concentration de Diltiazem (1562 nM) avec des concentrations en acide de Baloxavir proches de son IC50 (0.1 nM) ;
(ii) les conditions associant de plus faibles concentrations de Diltiazem (inférieures à 25 nM) avec de très faibles doses d'acide de Baloxavir (inférieure à 0.1 nM).

Dans toutes les conditions testées de concentrations en combinaison, aucun effet antagoniste n'a été observé.

Les résultats montrent que la combinaison du Diltiazem et de la forme active du Baloxavir permet une réduction très significative et synergique de la réplication virale (notamment dans des conditions d'utilisation de doses faibles de Baloxavir, ce qui est un avantage en termes de diminution du risque d'apparition de mutation de résistance au Baloxavir) dans les cellules A549 en comparaison avec un traitement en monothérapie avec chacune des deux molécules seules, aux mêmes concentrations.

### Exemple 2 (hors invention) - La combinaison Diltiazem + Favipiravir est synergique ou antagoniste, selon les concentrations de chacune des deux molécules, contre un virus recombinant H1N1nLuc en système cellulaire

Des cellules A549 infectées par A/H1N1nLuc (MOI 0,01) ont été traitées une heure post-infection par des concentrations croissantes de Diltiazem (6nM à 100µM) et de Favipiravir (6nM à 100µM) seul et en combinaison.

La luminescence mesurée dans les surnageants cellulaires d'infection (prélevés à 48 heures post-infection) reflète le niveau de réplication virale (chronogramme d'expérience représenté en Fig 2a).

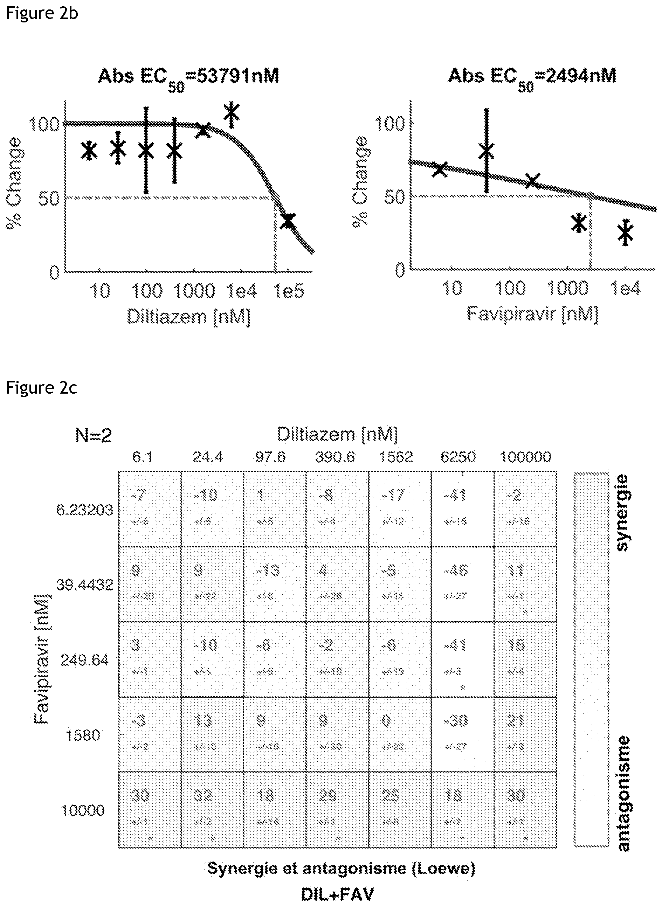

Les EC50 de chacune des molécules ont été déterminés (EC50 Diltiazem= 53791 nM ; EC50 Favipiravir= 2494 nM, voir Fig 2b).

Les effets de la combinaison des molécules ont été caractérisés par l'analyse via le logiciel Combenefit des données de luminescence mesurées (représentation sous forme d'un tableau avec une échelle chromatique, voir Fig 2c).

L'effet synergique ou antagoniste de la combinaison est représenté par le « coefficient de synergie » (Synergy Score), avec une échelle chromatique afin de faciliter l'interprétation des résultats. Un coefficient ≥ à 5 (gris à gris foncé) indique un effet synergique de la combinaison de molécules, tandis qu'un coefficient ≤ à -5 (blanc) indique un effet antagoniste de la combinaison de molécules. Des coefficients entre -5 et 5 sont indicatifs d'une « non-interference » des molécules dans la combinaison, voire d'effet additif entre les différents traitements. Nombre d'expériences = 3.

Les résultats obtenus montrent que la combinaison de ces deux molécules (Diltiazem + Favipiravir) peut améliorer l'effet antiviral mais, de façon générale, ne permettrait pas une réduction significative des doses de traitements.

En fonction de la concentration de chacune des molécules Diltiazem et Favipiravir, leurs combinaisons sont associées à des effets très hétérogènes. Pour obtenir un effet synergique, il est nécessaire d'utiliser des doses importantes de Favipiravir (supérieur à 1580 nM, ce qui est un désavantage puisqu'en faveur d'une augmentation du risque d'apparition de résistance au Favipiravir) avec différentes doses de Diltiazem (de 6 nM à 100 µM).

De plus, dans certaines conditions, l'effet de la combinaison des deux molécules peut être antagoniste. Ces effets antagonistes sont observés lorsque les doses de Diltiazem utilisées sont fortes (6250 nM).

La combinaison Diltiazem + Baloxavir permet d'obtenir des résultats homogènes même en changeant les concentrations utilisées, au contraire de la combinaison Diltiazem + Favipiravir qui n'est donc pas privilégiée.

### Exemple 3. Synergie de la combinaison Diltiazem + Baloxavir en modèle d'épithélium respiratoire humain

La réduction de la réplication des virus A/H1N1 et A/H3N2 est significativement plus importante avec le traitement en combinaison Diltiazem + Baloxavir (forme active) en comparaison des traitements en monothérapie avec du Diltiazem ou du Baloxavir seuls.

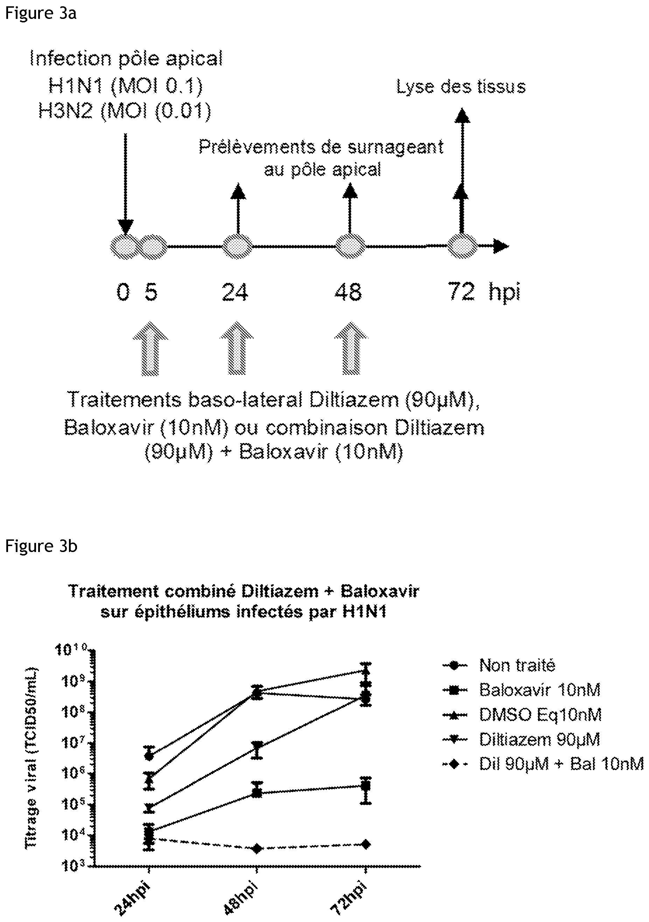

Des épithéliums respiratoires (d'origine nasale) humains reconstitués (MucilAir^{®} HAE, Epithelix) cultivés l'interface air-liquide (selon les instructions du fournisseur Epithelix), ont été infectés avec des virus influenza prototypes (non recombinants) de type A/H1N1 pdm09 (A/Lyon/969/2009 H1N1) (MOI 0.1) ou A/H3N2 (A/Texas/50/2012 H3N2) (MOI 0.01), puis traités ou non (non traité) par 3 doses successives de Diltiazem (90 µM), de Baloxavir (10 nM) ou d'une combinaison de Diltiazem (90 µM) et de Baloxavir (10 nM) délivrées à 5, 24 et 48 heures post-infection (hpi), respectivement (chronogramme d'expérience représenté en Fig 3a).

Des prélèvements au pôle apical des épithéliums infectés, traités ou non, ont été réalisés à 24, 48 et 72 hpi pour mesurer la réplication virale par titration infectieuse en TCID50/mL sur cellules MDCK.

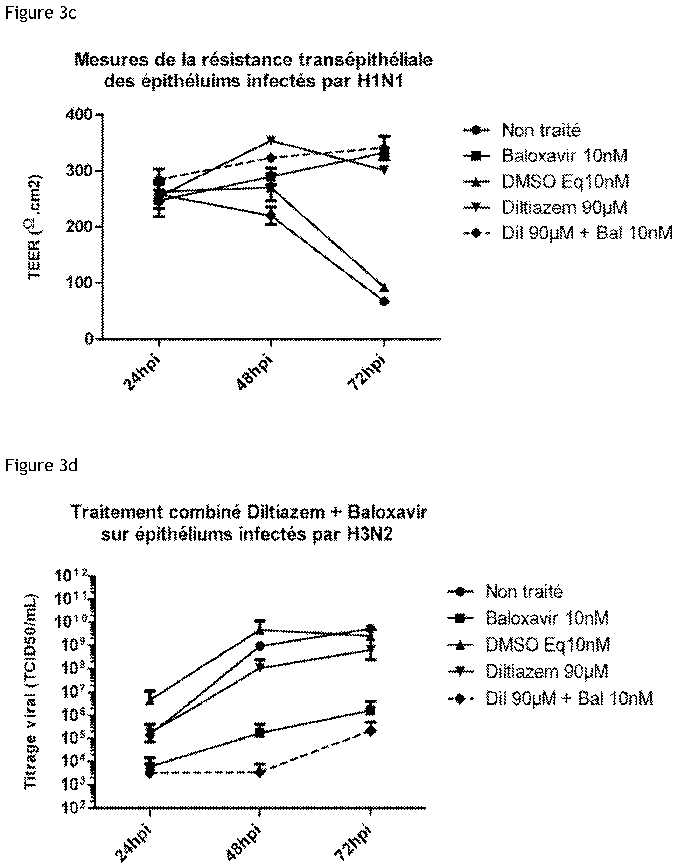

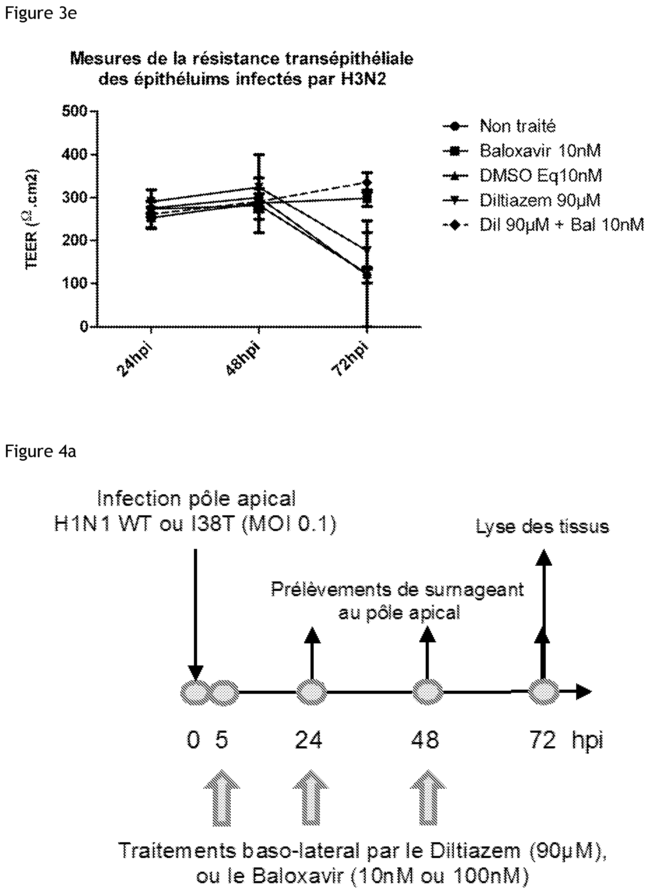

Avant chaque prélèvement au pôle apical, une mesure de la résistance trans-épithéliale (marqueur physiologique de l'intégrité des épithélium) a également été réalisée au moyen de l'appareil EVOM2 et de la sonde STX2 (World Precision Instruments) (Fig 3c et 3e).

Comme cela est illustré en figure 3b, sans traitement (non traité), l'infection virale induit une production de particules infectieuses A/H1N1 avec des titres supérieurs à 10⁸ TCID50/mL dès 48H post-infection.

Le traitement au Diltiazem (90 µM) permet de réduire la production de particules infectieuses A/H1N1 jusqu'à 2 log10 à 48H post-infection et d'au moins 1 log10 à 72H post-infection.

Le traitement au Baloxavir présente une activité antivirale in vitro importante avec une réduction jusqu'à 4 log10 de la production de particules infectieuses A/H1N1 à 48H post-infection, alors que son solvant seul (DMSO) n'a pas d'impact sur l'infection.

La combinaison des deux molécules Diltiazem et Baloxavir améliore significativement l'effet antiviral par rapport aux traitements en monothérapie, avec une réduction de la production virale A/H1N1 de près de 6log10 à 48 et 72H post-infection.

La figure 3c présente le suivi des mesures de résistance trans-épithéliale (TEER) au cours de l'infection des épithéliums par A/H1N1, tel que décrit en Fig 3a. Elle montre une chute importante de l'intégrité des épithéliums non traités ou traités par le solvant témoin DMSO à 48H et à 72H post-infection, en corrélation avec une infection très efficace, telle que présentée en Fig 3b.

En revanche, le traitement au Diltiazem permet de maintenir l'intégrité des épithéliums infectés avec des valeurs de TEER qui restent stables, en corrélation avec un effet antiviral démontré par titration infectieuse (Fig 3b).

Le traitement au Baloxavir permet également de maintenir l'intégrité des épithéliums infectés avec des mesures de valeurs de TEER similaires à celles obtenus avec le traitement au Diltiazem.

De la même manière, l'intégrité des épithéliums infectés est également maintenue avec le traitement en combinaison des deux molécules, en corrélation avec son efficacité antivirale (Fig 3b).

Ces mesures de TEER confirment également une absence d'effet cytotoxique du traitement par le Diltiazem et le Baloxavir en combinaison.

Comme cela est illustré en figure 3d, sans traitement (non traité), l'infection virale A/H3N2 des épithéliums induit une production de particules infectieuses avec des titres supérieurs à 10⁹ TCID50/mL dès 48H post-infection.

Le traitement au Diltiazem (90µM) permet de réduire la production de particules infectieuses A/H3N2 jusqu'à 2log10 à 48H post-infection et d'au moins 1log10 à 72H post-infection.

Le traitement au Baloxavir présente une activité antivirale in vitro importante avec une réduction jusqu'à 3log10 de la production de particules infectieuses A/H3N2 à 48H post-infection, alors que son solvant seul (DMSO) n'a pas d'impact sur l'infection.

La combinaison des deux molécules Diltiazem et Baloxavir améliore significativement l'effet antiviral par rapport aux traitements en monothérapie, avec une réduction de la production virale A/H3N2 de près de 5log10 à 48 et 4 log 10 à 72H post-infection.

Le suivi des mesures de résistance transépithéliale (TEER) au cours de l'infection des épithéliums par A/H3N2, tel que décrit en Fig 3a, présenté en Fig 3e, montre une chute importante de l'intégrité des épithéliums non traités ou traités par le solvant témoin DMSO à 48H et à 72H post-infection, en corrélation avec une infection très efficace, telle que mesurée et présentée en Fig 3d.

En revanche, le traitement au Diltiazem permet de maintenir l'intégrité des épithéliums infectés par A/H3N2 avec des valeurs de TEER qui restent stables jusqu'à 48H post-infection, en corrélation avec un effet antiviral démontré par titration infectieuse (Fig 3d).

Le traitement au Baloxavir permet également de maintenir l'intégrité des épithéliums infectés par A/H3N2 avec des mesures de valeurs de TEER similaires à celles obtenus avec le traitement au Diltiazem à 48H post-infection.

De la même manière, l'intégrité des épithéliums est également maintenue avec le traitement en combinaison des deux molécules, en corrélation avec son efficacité antivirale (Fig 3d).

Ces mesures de TEER confirment également une absence d'effet cytotoxique sur les épithéliums du traitement par le Diltiazem et le Baloxavir en combinaison.

En conclusion, les figures 3b et 3d démontrent un effet antiviral significatif des traitements par le Diltiazem ou le Baloxavir en comparaison de la condition d'infection sans traitement. Les résultats indiquent également l'effet bénéfique/synergique d'un traitement en combinaison Diltiazem + Baloxavir en comparaison des traitements en monothérapie avec chacune des deux molécules seules aux mêmes concentrations : la combinaison permet en effet une réduction significativement plus importante de la réplication virale en comparaison des traitements en monothérapies dans le modèle d'épithélium respiratoire humain.

### Exemple 4 (hors invention) - Le Diltiazem a une activité antivirale efficace contre un virus A/H1N1 recombinant I38T résistant au Baloxavir, en modèle d'épithélium respiratoire humain reconstitué, comparable à son activité antivirale contre le virus A/H1N1 recombinant sauvage (WT) dans le même modèle d'épithélium.

Le virus A/H1N1 résistant a été généré par génétique inverse. Il comporte la mutation de résistance I38T dans la sous-unité PA de sa polymérase.

Des épithéliums respiratoires humains reconstitués (MucilAir^{®} HAE, Epithelix) d'origine nasale, maintenus en culture en interface air-liquide selon les instructions du fournisseur Epithelix, ont été infectés avec des virus influenza recombinants de type A H1N1 pdm09 sauvage (WT, MOI 0.1) ou résistant au Baloxavir (mutation I38T sur la sous-unité PA de la polymérase virale) (138T, MOI 0.1).

Ces épithéliums ont ensuite été traités ou non (non traité) par 3 doses successives de Diltiazem (90 µM) seul, de Baloxavir seul à une concentration de 10 nM ou de 100 nM, ou par une combinaison des deux molécules (Diltiazem à 90 µM et Baloxavir à 10 nM ou 100 nM), délivrés à 5, 24 et 48 heures post-infection (hpi), respectivement (chronogramme d'expérience représenté en Fig 4a).

Des prélèvements au pôle apical des épithéliums infectés, traités ou non, ont été réalisés à 24, 48 et 72hpi pour mesurer la réplication virale par titration infectieuse en TCID50/mL sur cellules MDCK.

Avant chaque prélèvement au pôle apical, une mesure de la résistance trans-épithéliale (marqueur physiologique de l'intégrité des épithéliums) a également été réalisée au moyen de l'appareil EVOM2 et de la sonde STX2 (World Precision Instruments).

Chaque condition expérimentale a été réalisée en duplicata (n=2). Les graphes présentent des valeurs de moyennes et d'écart-types qui ont été générées via le logiciel GraphPad.

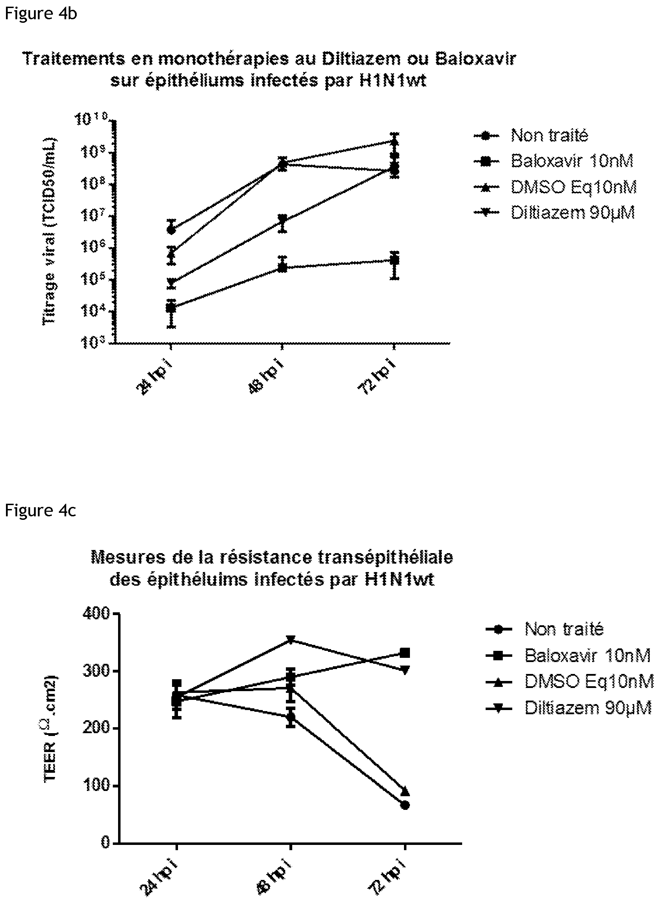

La figure 4b illustre le fait que sans traitement (non traité), l'infection virale induit une production de particules infectieuses A/H1N1 WT avec des titres supérieurs à 10⁸ TCID50/mL dès 48H post-infection.

Le traitement au Diltiazem (90 µM) permet de réduire la production de particules infectieuses A/H1N1 jusqu'à 2 log10 à 48H post-infection et 0.5log10 à 72H post-infection.

Le traitement au Baloxavir présente une activité antivirale in vitro importante avec une réduction jusqu'à 3 log10 de la production de particules infectieuses A/H1N1 à 48H post-infection, alors que son solvant seul (DMSO) n'a pas d'impact significatif sur l'infection.

Le suivi des mesures de résistance transépithéliale (TEER) au cours de l'infection, présenté en Fig. 4c, montre une chute importante de l'intégrité des épithéliums non traités ou traités par le solvant témoin DMSO à 48H et à 72H post-infection, en corrélation avec une infection très efficace, telle que présentée en Fig 4b.

En revanche, le traitement au Diltiazem permet de maintenir l'intégrité des épithéliums infectés par A/H1N1 avec des valeurs de TEER qui restent stables jusqu'à 48H post-infection, en corrélation avec un effet antiviral démontré par titration infectieuse (Fig 4b).

Le traitement au Baloxavir permet également de maintenir l'intégrité des épithéliums infectés par A/H1N1 avec des mesures de valeurs de TEER stables à 48H et 72H post-infection.

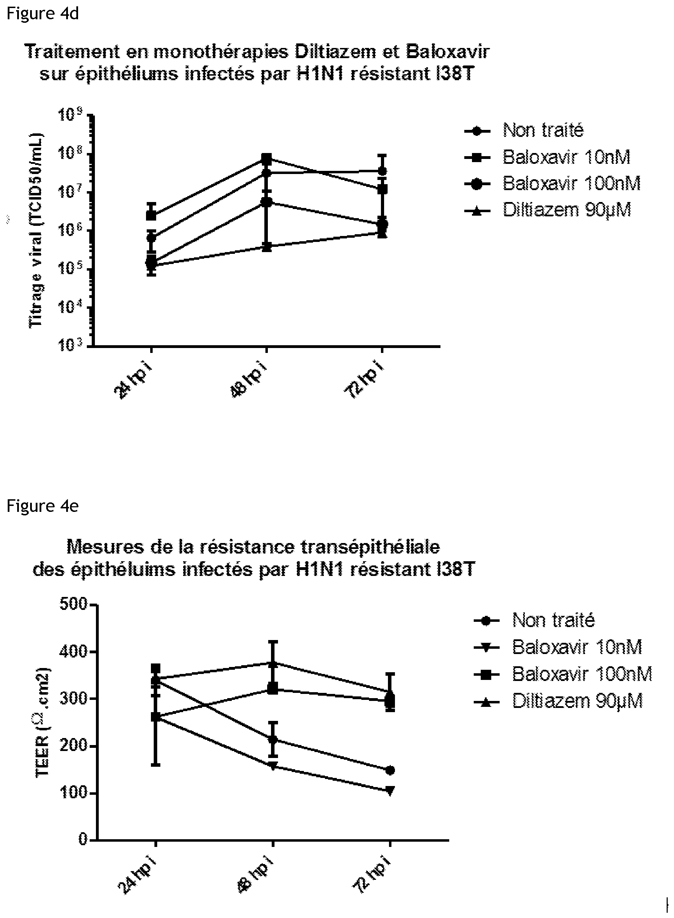

La figure 4d illustre le fait que sans traitement (non traité), l'infection des épithéliums avec le virus A/H1N1 I38T résistant au Baloxavir induit une production de particules infectieuses avec des titres proches de 10⁸ TCID50/mL à 48H post-infection (voir Fig 4d).

Le traitement au Baloxavir à 10 nM n'a pas d'effet antiviral sur ce virus résistant A/H1N1 I38T dans le modèle d'épithélium infecté. Il est nécessaire d'augmenter fortement la dose de traitement au Baloxavir (100 nM) pour obtenir une activité antivirale qui reste toutefois limitée contre ce virus résistant A/H1N1 I38T (réduction de 1 log10 de la production de particules infectieuses à 48H post-infection et de 1.5 log10 à 72H post-infection).

En revanche, le traitement au Diltiazem seul (90 µM) permet de réduire de manière significative la production de particules infectieuses A/H1N1 I38T de près de 2 log10 à 48H post-infection et de près de 2 log10 à 72H post-infection.

Le suivi des mesures de résistance trans-épithéliale (TEER) au cours de l'infection par A/H1N1 I38T, illustré en Fig 4e, montre une chute importante de l'intégrité des épithéliums non traités à 48H et à 72H post-infection, en corrélation avec une infection très efficace, telle que présentée en Fig 4d.

Au contraire, le traitement au Diltiazem permet de maintenir l'intégrité des épithéliums infectés par le virus A/H1N1 I38T avec des valeurs de TEER qui restent stables à 48H et 72H post-infection, en corrélation avec un effet antiviral démontré par titration infectieuse (Fig 4d).

Par contre, le traitement au Baloxavir à 10 nM ne permet pas de maintenir l'intégrité des épithéliums infectés par A/H1N1 I38T à 48H et 72H post-infection, en cohérence avec l'absence d'activité antivirale telle rapportée dans la Fig 4d. Seul le traitement à dose forte de Baloxavir (100 nM), pour lequel une activité antivirale significative contre le virus résistant A/H1N1 I38T a été rapportée dans la Fig 4d, permet le maintien de l'intégrité de l'épithélium, telle que rapportée par les mesures de valeurs stables de TEER à 48H et 72H post-infection.

### Exemple 5 - La combinaison Diltiazem + Baloxavir induit une réduction significativement plus importante de la réplication du virus A/H1N1 I38T résistant au Baloxavir en comparaison des traitements en monothérapie, mais uniquement lorsque le Baloxavir est utilisé à forte dose.

Des épithéliums respiratoires humains reconstitués (MucilAir^{®} HAE, Epithelix) d'origine nasale, maintenus en culture en interface air-liquide selon les instructions du fournisseur Epithelix, ont été infectés avec un virus influenza recombinant de type A H1N1 pdm09 résistant au Baloxavir (mutation I38T sur la sous-unité PA de la polymérase virale) (138T, MOI 0.1).

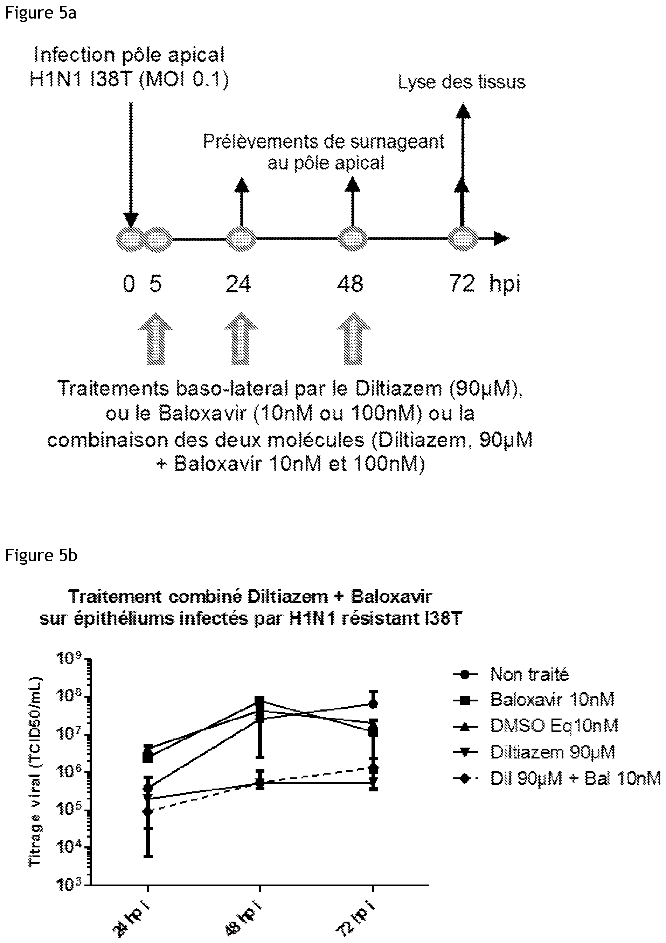

Ces épithéliums ont ensuite été traités ou non (non traité) par 3 doses successives de Diltiazem (90 µM) seul, de Baloxavir seul à une concentration de 10 nM ou de 100 nM, ou par une combinaison des deux molécules (Diltiazem à 90 µM et Baloxavir à 10 nM ou 100 nM), délivrés à 5, 24 et 48 heures post-infection (hpi), respectivement (chronogramme d'expérience représenté en Fig 5a).

Des prélèvements au pôle apical des épithéliums infectés, traités ou non, ont été réalisés à 24, 48 et 72hpi pour mesurer la réplication virale par titration infectieuse en TCID50/mL sur cellules MDCK.

Avant chaque prélèvement au pôle apical, une mesure de la résistance trans-épithéliale (marqueur physiologique de l'intégrité des épithéliums) a également été réalisée au moyen de l'appareil EVOM2 et de la sonde STX2 (World Precision Instruments).

La figure 5b montre que, sans traitement (non traité), l'infection des épithéliums par le virus A/H1N1 I38T résistant au Baloxavir induit une production de particules infectieuses avec des titres à près de 10⁸ TCID50/mL à 72H post-infection. Le traitement au Diltiazem (90µM) permet de réduire la production de particules infectieuses A/H1N1 I38T jusqu'à 2log10 à 48H post-infection et d'au moins 2log10 à 72H post-infection.

En revanche, le traitement au Baloxavir (10 nM) est inefficace tout comme son solvant seul (DMSO) qui n'a pas d'impact sur l'infection.

La combinaison des deux molécules Diltiazem et Baloxavir à leur concentration respective permet de réduire la production de particules infectieuses A/H1N1 I38T mais sans effet supplémentaire ou synergique par rapport au traitement avec le Diltiazem seul à 48H et 72H post-infection.

Ce résultat est différent de celui obtenu sur le virus A/H1N1 WT (non résistant au Baloxavir, cf Fig 3b) sur lequel la combinaison des deux molécules Diltiazem et Baloxavir aux mêmes concentrations a un effet antiviral synergique par rapport aux traitements en monothérapie, en induisant notamment une réduction de la production virale de près de 6log10 à 48 et 72H post-infection.

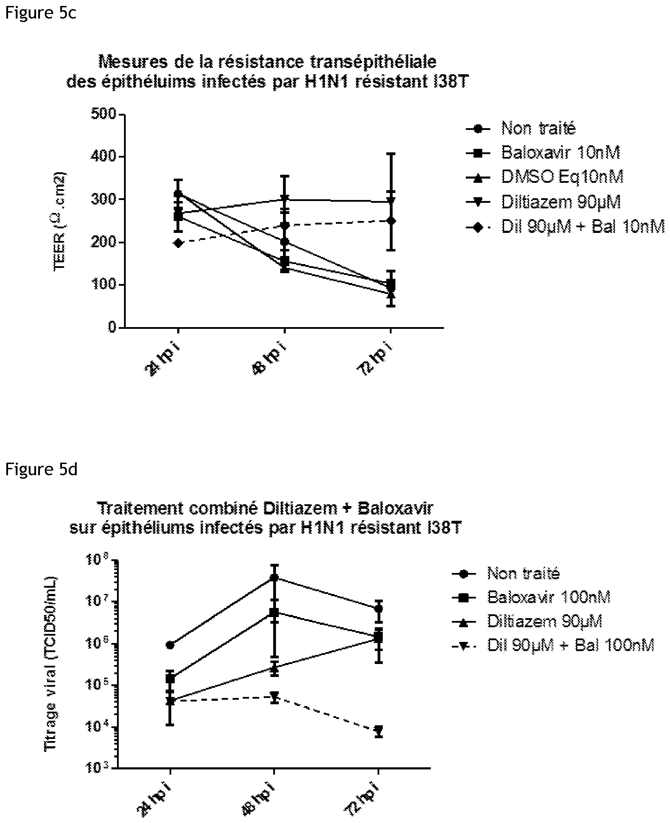

Le suivi des mesures de résistance trans-épithéliale (TEER) au cours de l'infection, présenté en Fig 5c, montre une chute importante de l'intégrité des épithéliums non traités ou traités par le solvant témoin DMSO à 48H et à 72H post-infection, en corrélation avec une infection très efficace, telle que mesurée et présentée en Fig 5b.

En revanche, le traitement au Diltiazem permet de maintenir l'intégrité des épithéliums infectés par A/H1N1 I38T avec des valeurs de TEER qui restent stables à 48H et 72H post-infection, en corrélation avec un effet antiviral démontré par titration infectieuse (Fig 5b).

Le traitement au Baloxavir (10 nM) ne permet pas de maintenir l'intégrité des épithéliums infectés par A/H1N1 I38T en cohérence avec son inefficacité antivirale, telle que rapportée dans Fig 5b.

En cohérence également avec son efficacité antivirale significative, la combinaison Diltiazem + Baloxavir est associée à des mesures de valeurs de TEER stables à 48H et 72H post-infection.

La figure 5d illustre que, sans traitement (non traité), l'infection des épithélium par le virus A/H1N1 I38T résistant au Baloxavir induit une production de particules infectieuses avec des titres à près de 10⁸ TCID50/mL à 48H post-infection.

Le traitement au Diltiazem (90 µM) permet de réduire la production de particules infectieuses A/H1N1 I38T à plus de 2log10 à 48H post-infection.

Utilisé à une concentration forte (100 nM), le traitement au Baloxavir réduit d'environ 1log10 la production de particules infectieuses A/H1N1 I38T à 48H post-infection, alors que le traitement à une concentration de 10 nM n'avait aucun effet sur le virus résistant I38T (cf Fig 5b).

La combinaison des deux molécules Diltiazem et Baloxavir aux mêmes concentrations respectives permet de réduire davantage la production de particules infectieuses A/H1N1 I38T à 48H et surtout à 72H post-infection (avec une réduction jusqu'à 3log10 de particules infectieuses A/H1N1 I38T) en comparaison aux traitements en monothérapie. Le gain de la combinaison du Diltiazem avec le Baloxavir (à forte concentration=100 nM) est donc significatif contre le virus résistant A/H1N1 I38T dans le modèle d'épithélium respiratoire humain.

Cette combinaison est particulièrement pertinente contre des souches virales résistantes au Baloxavir, contre lesquelles il est nécessaire d'augmenter significativement les doses de Baloxavir.

Le suivi des mesures de résistance trans-épithéliale (TEER) au cours de l'infection, présenté en Fig 5e, montre une chute importante de l'intégrité des épithéliums non traités à 48H et à 72H post-infection, en corrélation avec une infection très efficace, telle que mesurée et présentée en Fig 5d.

En revanche, les traitements au Diltiazem ou au Baloxavir à forte dose (100 nM) permettent de maintenir l'intégrité des épithéliums infectés par A/H1N1 I38T avec des valeurs de TEER qui restent stables à 48H et 72H post-infection, en corrélation avec des effets antiviraux démontrés par titration infectieuse (Fig 5d).

En cohérence également avec son efficacité antivirale significative (cf Fig 5d), la combinaison Diltiazem + Baloxavir est associée à des mesures de valeurs de TEER stables à 48H et 72H post-infection.

En conclusion, les résultats obtenus et présentés dans les exemples 4 et 5 :
(i) confirment le phénotype de résistance au Baloxavir du virus recombinant A/H1N1 I38T (mutation I38T dans sa sous-unité PA de la polymérase), contre laquelle le traitement de référence au Baloxavir à 10 nM n'a aucune efficacité (Fig 4d).
(ii) La Fig 4d démontre également qu'il est nécessaire d'augmenter fortement la dose de traitement au Baloxavir (100 nM) pour obtenir une activité antivirale, qui reste toutefois limitée contre ce virus résistant A/H1N1 I38T.
(iii) De manière importante, les résultats indiquent également que le Diltiazem a une activité antivirale significativement efficace contre un virus A/H1N1 recombinant I38T résistant au Baloxavir en modèle d'épithélium respiratoire humain reconstitué, en comparaison de son activité antivirale contre le virus A/H1N1 recombinant sauvage (WT) dans le même modèle d'épithélium (comparer Fig 4b et 4d).
(iv) Les résultats présentés en Fig 5b confirment de nouveau que le traitement combiné du Diltiazem (90 µM) avec du Baloxavir (10 nM) permet une réduction significative de la réplication virale dans les épithéliums respiratoires de voies aériennes supérieures infectés par une souche sauvage de A/H1N1.
(v) En revanche, cette combinaison Diltiazem (90 µM) + Baloxavir (10 nM) n'apporte pas de gain supplémentaire par rapport à un traitement au Diltiazem en monothérapie contre le virus résistant A/H1N1 I38T (Fig 5b).
(vi) C'est en combinaison avec du Baloxavir à 100 nM que le Diltiazem (90 µM) apporte un gain significatif en termes d'activité antivirale contre le virus résistant A/H1N1 I38T en comparaison aux traitements en monothérapie de chacune des deux molécules aux mêmes concentrations dans le même modèle d'épithelium respiratoire humain (Fig 5d).

### Exemple 6 - L'utilisation du Diltiazem en combinaison au Baloxavir permet d'empêcher l'émergence de virus résistant au Baloxavir dans un test classique de passages cellulaires successifs de virus sous pression de sélection antivirale (concentration croissante de Baloxavir)

Des cellules MDCK en plaque de 24 puits ont été infectées par le virus A/H1N1 prototype WT (pour lequel les concentrations médianes inhibitrices IC50 de Baloxavir et de Diltiazem sont de 0.2 nM et 5 µM, respectivement) à une MOI de 0.001, constituant ainsi le premier passage cellulaire du virus (P0).

Quatre bras différents de passages cellulaires successifs de ce virus ont été menés dans des conditions de pression de sélection ou sans pression de sélection, telles que définies ci-après :
(i) sans traitement (NT),
(ii) traitement au Diltiazem (Dil) à une concentration fixe de 25 µM,
(iii) traitement au Baloxavir à des concentrations croissantes de 1 nM jusqu'à 128 nM et
(iv) traitement au Diltiazem (Dil) à une concentration fixe de 25 µM en combinaison avec du Baloxavir aux mêmes concentrations croissantes de 1 nM jusqu'à 128 nM que précédemment en (iii).

Dès le passage P0, les cellules ont été traitées à 1H post-infection selon les différentes conditions décrites précédemment ou non traitées (NT) et les surnageants d'infections ont été prélevés à 48H post-infection (J+2). Ces prélèvements infectieux ont été dilués en cascades (de 10-1 à 10-6) et chaque dilution a été déposée sur des cellules MDCK en plaque de 24 puits, constituant ainsi le passage cellulaire (P1) suivant le passage P0.

Une heure après cette infection, les cellules ont été traitées selon les différentes conditions décrites précédemment ou non traitées (NT). A l'issue de ce passage cellulaire P1 (48H post-infection, J+4), et pour chacun des bras de traitement (i, ii, iii et iv), il a été prélevé le surnageant du puits infecté avec la dilution dite « limite », c'est à dire la dernière des dilutions de 10-1 à 10-6 issues de P0 où il a été observé des effets cytopathiques (ECP) significatifs.

Ces surnageants d'infections ont été dilués en cascades (de 10-1 à 10-6) et chaque dilution a été déposée sur des cellules MDCK en plaque de 24 puits, constituant ainsi le passage cellulaire (P2) suivant le passage P1.

La procédure a ainsi été suivie pour chaque passage cellulaire successif. La concentration de Diltiazem est restée fixe pendant toute la durée de l'expérimentation. Celle du Baloxavir a été fixée à 1nM pendant les deux premiers passages (P0 et P1), puis a été doublée pendant les deux passages suivants (P3 et P4), avant d'être doublée à chaque passage jusqu'à 128 nM au dixième passage (cf Chronogramme de l'expérience Fig 6a).

A la fin de l'expérimentation, les surnageants infectieux issus du dernier passage cellulaire de chaque bras de traitement présentant des ECP significatifs ont été titrés en TCID50/mL sur cellules MDCK. La concentration médiane inhibitrice du Baloxavir sur les virus de ces surnageants a également été calculée par une méthode de titration en dilution limite en TCID50/mL sur cellules MDCK, afin de caractériser l'apparition d'un phénotype de résistance au Baloxavir en comparaison au virus sauvage initial utilisé.

Pour les bras expérimentaux non traités et de traitement au Diltiazem, ce sont les surnageants infectieux issus du 10^{ème} passage qui ont été utilisés. Pour le bras de traitement au Baloxavir, c'est le surnageant infectieux issu du 8^{ème} passage cellulaire qui a été utilisé. Pour le bras de traitement en combinaison Diltiazem + Baloxavir, c'est le surnageant infectieux issu du 6^{ème} passage cellulaire qui a été utilisé (car les virus ont été perdus aux 7^{ème} et 9^{ème} passages cellulaires pour ces bras expérimentaux, respectivement, cf Fig 6a et 6b).

Des cellules MDCK ont ainsi été infectées par les surnageants infectieux en question, puis traitées séparément une heure post infection par différentes concentrations croissantes de Baloxavir (0.04 nM à 160 nM). Parallèlement, des cellules MDCK ont aussi été infectées par le virus A/H1N1 WT utilisé initialement pour le passage cellulaire P0 (cf Fig 6a et 6b) à une MOI 0.001, puis traitées ou non séparément une heure post infection par différentes concentrations croissantes de Baloxavir (0.04 nM à 160 nM).

Des surnageants d'infection ont été prélevés 48hpi puis la charge virale a été titrée par la méthode en dilution limite en TCID50/mL sur cellules MDCK. Les résultats ont été exprimés en valeurs relatives par rapport aux cellules témoin infectées par le virus A/H1N1 et non traitées (Fig 6c à 6g). A partir de ces résultats de titrations virales infectieuses, les concentrations médianes inhibitrices de Baloxavir ont ainsi été calculées et sont rapportées dans le tableau 1 ci-dessous.

### Présentation détaillée des résultats

Le graphique de la figure 6b représente les concentrations utilisées de Baloxavir et de Diltiazem dans les différents bras expérimentaux (non traité, traitement au Baloxavir, traitement au Diltiazem et traitement en combinaison Diltiazem + Baloxavir) pour chaque passage cellulaire, selon le protocole expérimental décrit précédemment en Fig 6a. Le tableau présente les dilutions limites des surnageants infectieux issus de chaque passage cellulaire (pour lesquelles il a été observé des effets cytopathiques significatifs) et qui ont été utilisées pour infecter les cellules MDCK de chaque passage cellulaire suivant dans chacun des quatre bras expérimentaux (non traité, traitement au Baloxavir, traitement au Diltiazem et traitement en combinaison Diltiazem + Baloxavir).

Les bras expérimentaux sans traitement (non traité) et avec traitement au Diltiazem ont été maintenus sur les 10 passages cellulaires. Pour le bras sans traitement, la dilution limite utilisée pour chaque passage cellulaire a toujours été celle à 10⁻⁶. Pour le bras traité au Diltiazem, la dilution limite utilisée a varié entre 10⁻⁵ et 10⁻⁶, tel qu'indiqué dans le graphique et le tableau.

En ce qui concerne le bras avec le traitement au Baloxavir, la dilution limite utilisée pour chaque passage cellulaire est restée stable à 10⁻⁵ jusqu'au 6^{ème} passage cellulaire, pour lequel la concentration de Baloxavir utilisée était de 8 nM. A partir de ce passage cellulaire, la dilution limite utilisée pour les passages cellulaires suivants a diminué progressivement (de 10⁻⁴ à 10⁻¹) au fur et à mesure de l'utilisation de concentrations croissantes de Baloxavir (de 8nM jusqu'à 32nM) jusqu'à la perte du virus réplicatif au 9^{ème} passage cellulaire, pour lequel la concentration de Baloxavir utilisée était de 64nM.

Dans le bras de traitement par combinaison Diltiazem + Baloxavir, les dilutions limites utilisées (pour lesquelles il a été observé des effets cytopathiques significatifs) ont été plus faibles dès le premier passage cellulaire (10⁻³) et sont restées stables entre 10⁻³ et 10⁻² jusqu'au 6^{ème} passage cellulaire. La perte du virus réplicatif a été constatée lors du 7^{ème} passage cellulaire pour lequel les concentrations utilisées de Baloxavir et de Diltiazem étaient de 16 nM et 25 µM, respectivement.

Ces résultats indiquent que dans le bras de traitement au Baloxavir, le virus A/H1N1, pour lequel la concentration médiane inhibitrice (IC50) initiale de Baloxavir était de 0,2 nM, s'est répliqué jusqu'au 8ème passage cellulaire jusqu'à des conditions de concentration de Baloxavir de 32 nM. Ces résultats suggèrent que la pression de sélection exercée par le Baloxavir dans ces conditions expérimentales ait induit un phénotype viral de résistance. En revanche, ce phénotype de résistance virale n'a pas été observé dans le bras de traitement au Diltiazem, puisque tout comme dans le bras non traité, la dilution limite utilisée pour chaque passage cellulaire est restée stable entre 10⁻⁵ et 10⁻⁶, sans perte de virus jusqu'au 10^{ème} passage cellulaire.

Ces résultats indiquent également que l'ajout de Diltiazem au Baloxavir dans le bras de traitement par combinaison (Diltiazem 25µM + Baloxavir à des concentrations croissantes de 1 nM à 8 nM) a apporté un effet synergique d'inhibition virale en comparaison aux traitements seuls au Diltiazem ou au Baloxavir aux mêmes concentrations, puisque (i) les dilutions limites utilisées à chaque passage cellulaire ont toujours été plus faibles dans ce bras expérimental et ce dès le premier passage cellulaire (10⁻³), et (ii) le virus réplicatif a été perdu plus rapidement (dès le 7^{ème} passage cellulaire).

La figure 6c rapporte la détermination de la concentration médiane inhibitrice du Baloxavir sur le virus A/H1N1 issu du passage cellulaire initial P0. L'IC50 déterminée initialement sur le virus A/H1N1 utilisé pour l'expérimentation, est confirmée à 0.2 nM.

La figure 6d rapporte la détermination de la concentration médiane inhibitrice du Baloxavir sur le virus issu du passage cellulaire P10 du bras expérimental non traité. L'IC50 calculée du Baloxavir sur le virus A/H1N1 issu du 10ème passage cellulaire sans traitement (non traité), est à 0.9 nM. Cette légère augmentation peut être attribuée à une réplication virale du virus A/H1N1 qui s'est adaptée à l'amplification cellulaire successive sur cellules MDCK dans ce bras expérimental.

La figure 6e rapporte la détermination de la concentration médiane inhibitrice du Baloxavir sur le virus issu du passage cellulaire P10 du bras expérimental de traitement au Diltiazem. L'IC50 calculée du Baloxavir sur le virus A/H1N1 issu du 10ème passage cellulaire avec traitement au Diltiazem, est à 0.3 nM. Cette IC50 est sensiblement similaire à celle déterminée initialement sur le virus A/H1N1 utilisé pour l'expérimentation. Ce résultat confirme que le traitement au Diltiazem à une concentration constante de 25µM sur 10 passages cellulaires successifs ne modifie pas la sensibilité du virus A/H1N1 au Baloxavir (et n'induite pas l'émergence de virus au phénotype de résistance au Baloxavir).

La figure 6f rapporte la détermination de la concentration médiane inhibitrice du Baloxavir sur le virus issu du passage cellulaire P8 du bras expérimental de traitement au Baloxavir.

L'IC50 calculée du Baloxavir sur le virus A/H1N1 issu du 8ème passage cellulaire avec traitement au Baloxavir (en concentration croissante), est à 7 nM. Cette IC50 est très supérieure à celle déterminée initialement sur le virus A/H1N1 utilisé pour l'expérimentation (IC50=0,2nM).

Cette augmentation, correspondant à 35 fois l'IC50 initiale, confirme l'émergence d'un virus au phénotype de résistance au Baloxavir, en cohérence avec les observations décrites en Fig 6b (dilutions limites utilisées plus faibles dès le premier passage cellulaire dans ce bras expérimental, réplication virale effective jusqu'à des conditions de concentration de Baloxavir de 32 nM au 8ème passage cellulaire).

La figure 6g rapporte la détermination de la concentration médiane inhibitrice du Baloxavir sur le virus issu du passage cellulaire P6 du bras expérimental de traitement par combinaison Diltiazem + Baloxavir.

L'IC50 calculée du Baloxavir sur le virus A/H1N1 issu du 6ème passage cellulaire avec traitement par combinaison au Diltiazem + Baloxavir (en concentration croissante), est à 0,8 nM. Cette IC50 est très similaire à celle calculée pour le Baloxavir sur le virus A/H1N1 issu du 10ème passage cellulaire dans le bras expérimental sans traitement (0.9 nM, cf Fig 6d) et légèrement supérieure à celle calculée initialement sur le virus A/H1N1 utilisé pour l'expérimentation et à l'issue du passage cellulaire P0 (IC50=0,2 nM, cf Fig 6c). Outre que cette légère augmentation peut être attribuée à une réplication virale du virus A/H1N1 qui s'est adaptée à l'amplification cellulaire successive sur cellules MDCK dans ce bras expérimental, il est clairement établi que cette IC50 est très nettement inférieure à celle calculée pour le virus A/H1N1 issu du passage cellulaire P8 du bras expérimental de traitement au Baloxavir (IC50=7 nM, cf Fig 6f), indiquant l'absence d'émergence de virus au phénotype de résistance au Baloxavir dans ce bras expérimental. Ces résultats indiquent que l'utilisation du Diltiazem en combinaison au Baloxavir permet d'empêcher l'émergence de virus résistant au Baloxavir dans un test classique de passages cellulaires successifs de virus sous pression de sélection antivirale (concentration croissante de Baloxavir).

Les concentrations médianes inhibitrices de Baloxavir pour les virus A/H1N1 issus des derniers passages cellulaires des différents bras expérimentaux sont présentées dans le tableau 1 ci-dessous.

**Tableau 1**

| **Bras expérimental** | **IC50 Baloxavir (forme active)** |
|---|---|
| H1N1 WT Initial PO | 0.2 nM |
| H1N1 NT P10 | 0.9 nM |
| H1N1 Dil P10 | 0.3 nM |
| H1N1 Baloxavir marboxil P8 | 7 nM |
| H1N1 Combinaison P6 | 0.8 nM |

En conclusion, les résultats de cette expérimentation de passages cellulaires successifs en cellules MDCK de virus A/H1 N1 en conditions de pression de sélection, indiquent que, comme attendu, l'utilisation de concentrations croissantes de Baloxavir a induit l'émergence d'un virus A/H1N1 au phénotype de résistance au Baloxavir, alors que dans les mêmes conditions de concentration croissante de Baloxavir, l'ajout en combinaison de Diltiazem à une concentration constante de 25 µM permet d'empêcher l'émergence d'un virus A/H1N1 au phénotype de résistance au Baloxavir.

De manière similaire et comme attendu, la condition expérimentale sans traitement n'a pas non plus induit l'émergence d'un virus A/H1N1 au phénotype de résistance au Baloxavir.

De même, la condition expérimentale avec traitement au Diltiazem seul n'a pas non plus induit l'émergence d'un virus A/H1N1 au phénotype de résistance au Baloxavir.

### REFERENCES BIBLIOGRAPHIQUES dans l'ordre de citation dans la description

### BREVETS

WO 87/07508
WO 2011/066657
WO 2016/146836
WO 2019/224489
WO 02/094238
US 4,605,552

### ARTICLES

Pizzorno A, Terrier O, Nicolas de Lamballerie C, Julien T, Padey B, Traversier A, Roche M, Hamelin ME, Rhéaume C, Croze S, Escuret V, Poissy J, Lina B, Legras-Lachuer C, Textoris J, Boivin G, Rosa-Calatrava M. Repurposing of Drugs as Novel Influenza Inhibitors From Clinical Gene Expression Infection Signatures. Front Immunol. 2019 Jan 29;10:60.
Pizzorno A, Padey B, Terrier O, Rosa-Calatrava M. Drug Repurposing Approaches for the Treatment of Influenza Viral Infection: Reviving Old Drugs to Fight Against a Long-Lived Enemy. Front Immunol. 2019 Mar 19;10:531. (Review)
Wang W, Shin WJ, Zhang B, Choi Y, Yoo JS, Zimmerman MI, Frederick TE, Bowman GR, Gross ML, Leung DW, Jung JU, Amarasinghe GK. The Cap-Snatching SFTSV Endonuclease Domain Is an Antiviral Target. Cell Rep. 2020 Jan 7;30(1):153-163.e5.
Omoto S, Speranzini V, Hashimoto T, Noshi T, Yamaguchi H, Kawai M, Kawaguchi K, Uehara T, Shishido T, Naito A, Cusack S. Characterization of influenza virus variants induced by treatment with the endonuclease inhibitor baloxavir marboxil. Sci Rep. 2018 Jun 25;8(1):9633.
Uehara T, Hayden FG, Kawaguchi K, Omoto S, Hurt AC, De Jong MD, Hirotsu N, Sugaya N, Lee N, Baba K, Shishido T, Tsuchiya K, Portsmouth S, Kida H. Treatment-Emergent Influenza Variant Viruses With Reduced Baloxavir Susceptibility: Impact on Clinical and Virologic Outcomes in Uncomplicated Influenza. J Infect Dis. 2019 Jul 16.
Takashita E, Kawakami C, Ogawa R, Morita H, Fujisaki S, Shirakura M, Miura H, Nakamura K, Kishida N, Kuwahara T, Ota A, Togashi H, Saito A, Mitamura K, Abe T, Ichikawa M, Yamazaki M, Watanabe S, Odagiri T. Influenza A(H3N2) virus exhibiting reduced susceptibility to baloxavir due to a polymerase acidic subunit I38T substitution detected from a hospitalised child without prior baloxavir treatment, Japan, January 2019. Euro Surveill. 2019 Mar;24(12).
Hayden FG, Shindo N. Influenza virus polymerase inhibitors in clinical development. Curr Opin Infect Dis. 2019 Apr;32(2):176-186
Fukao K, Noshi T, Yamamoto A, Kitano M, Ando Y, Noda T, Baba K, Matsumoto K, Higuchi N, Ikeda M, Shishido T, Naito A. Combination treatment with the cap-dependent endonuclease inhibitor baloxavir marboxil and a neuraminidase inhibitor in a mouse model of influenza A virus infection. J Antimicrob Chemother. 2019 Mar 1;74(3):654-662.
Hayden FG, Sugaya N, Hirotsu N, Lee N, de Jong MD, Hurt AC et al. Baloxavir Marboxil for Uncomplicated Influenza in Adults and Adolescents. N Engl J Med, 2018 Sep 6;379(10):913-23
Noshi T, Kitano M, Taniguchi K, Yamamoto A, Omoto S, Baba K, et al. In vitro characterization of baloxavir acid, a first-in-class cap-dependent endonuclease inhibitor of the influenza virus polymerase PA subunit. Antiviral Res. 2018;160:109-17.
Clark MP, Ledeboer MW, Davies I, Byrn RA, Jones SM, Perola E, et al. Discovery of a novel, first-in-class, orally bioavailable azaindole inhibitor (VX-787) of influenza PB2. J Med Chem. 2014;57(15):6668-78.
Trevejo JM, Asmal M, Vingerhoets J, Polo R, Robertson S, Jiang Y, et al. Pimodivir treatment in adult volunteers experimentally inoculated with live influenza virus: a Phase IIa, randomized, double-blind, placebo-controlled study. Antivir Ther. 2018;23(4):335G44
Yousuke Furuta, Kazumi Takahashi, Kimiyasu Shiraki, Kenichi Sakamoto, Donald F. Smee, Dale L. Barnard, Brian B. Gowen, Justin G. Julander et John D. Morrey, « T-705 (favipiravir) and related compounds: Novel broad-spectrum inhibitors of RNA viral infections », Antiviral Research, vol. 82, no 3, juin 2009, p. 95-102.
Goldhill DH, Te Velthuis AJW, Fletcher RA, Langat P, Zambon M, Lackenby A, Barclay WS. The mechanism of resistance to favipiravir in influenza. Proc Natl Acad Sci U S A. 2018 Nov 6;115(45):11613-11618.

## Revendications

1. Combinaison de Diltiazem et de Baloxavir marboxil, pour son utilisation dans la prévention et/ou le traitement d'une infection virale par un virus influenza.

2. Combinaison pour son utilisation selon la revendication 1, **caractérisée en ce que** ledit virus influenza est résistant à l'action inhibitrice d'au moins un composé antiviral, notamment d'un composé anti-grippal, et plus particulièrement d'un composé inhibiteur de polymérase virale.

3. Combinaison pour son utilisation selon l'une des revendications 1 ou 2, **caractérisée en ce que** ladite combinaison comprend au moins un autre agent actif, notamment un agent antiviral et/ou un antibiotique.

4. Composition pharmaceutique comprenant, dans un véhicule pharmaceutique, une combinaison de Diltiazem et de Baloxavir marboxil, pour son utilisation thérapeutique dans la prévention et/ou le traitement d'une infection virale par un virus influenza..

5. Composition pharmaceutique pour son utilisation selon la revendication 4, **caractérisée en ce qu'**elle comprend de plus un autre agent actif, notamment un agent antiviral et/ou un antibiotique.

6. Composition pharmaceutique pour son utilisation selon l'une des revendications 4 ou 5, **caractérisée en ce que** ladite composition est sous une forme galénique adaptée pour une administration par inhalation.

7. Produit de combinaison comprenant du Diltiazem et du Baloxavir marboxil, pour son utilisation simultanée, séparée ou séquentielle dans la prévention et/ou le traitement d'une infection virale par un virus influenza.

## Patentansprüche

1. Kombination aus Diltiazem und Baloxavir marboxil für ihre Verwendung bei der Vorbeugung und/oder der Behandlung einer Virusinfektion durch ein Influenzavirus.

2. Kombination für ihre Verwendung nach Anspruch 1, **dadurch gekennzeichnet, dass** das Influenzavirus gegen die hemmende Wirkung von mindestens einer antiviralen Verbindung, insbesondere einer Verbindung gegen Grippe, und insbesondere einer Verbindung, die die virale Polymerase hemmt, resistent ist.

3. Kombination für ihre Verwendung nach einem der Ansprüche 1 oder 2, **dadurch gekennzeichnet, dass** die Kombination mindestens einen weiteren Wirkstoff, insbesondere einen antiviralen Wirkstoff und/oder ein Antibiotikum, umfasst.

4. Pharmazeutische Zusammensetzung, die in einem pharmazeutischen Träger eine Kombination aus Diltiazem und Baloxavir marboxil für ihre therapeutische Verwendung bei der Vorbeugung und/oder der Behandlung einer Virusinfektion durch ein Influenzavirus umfasst.

5. Pharmazeutische Zusammensetzung für ihre Verwendung nach Anspruch 4, **dadurch gekennzeichnet, dass** sie zusätzlich einen weiteren Wirkstoff, insbesondere einen antiviralen Wirkstoff und/oder ein Antibiotikum, umfasst.

6. Pharmazeutische Zusammensetzung für ihre Verwendung nach einem der Ansprüche 4 oder 5, **dadurch gekennzeichnet, dass** die Zusammensetzung in einer galenischen Form vorliegt, die für eine Verabreichung durch Inhalation geeignet ist.

7. Kombinationsprodukt, umfassend Diltiazem und Baloxavir marboxil, für ihre gleichzeitige, getrennte oder sequentielle Verwendung bei der Vorbeugung und/oder der Behandlung einer Virusinfektion durch ein Influenzavirus.

## Claims

1. Combination of Diltiazem and Baloxavir marboxil, for use in the prevention and/or treatment of viral infection by an influenza virus.

2. Combination for use according to claim 1, **characterized in that** said influenza virus is resistant to the inhibitory action of at least one antiviral compound, in particular an anti-influenza compound, and more particularly a viral polymerase inhibitor compound.

3. A combination for use according one of claims 1 or 2, **characterised in that** said combination comprises at least one other active agent, in particular an antiviral agent and/or an antibiotic.

4. Pharmaceutical composition comprising, in a pharmaceutical vehicle, a combination of Diltiazem and Baloxavir marboxil, for its therapeutic use in the prevention and/or treatment of a viral infection by an influenza virus.

5. Pharmaceutical composition for use according to claim 4, **characterized in that** it further comprises another active agent, in particular an antiviral agent and/or an antibiotic.

6. Pharmaceutical composition for use according to one of claims 4 or 5, **characterised in that** said composition is in a galenic form suitable for administration by inhalation.

7. Combination product comprising Diltiazem and Baloxavir marboxil, for simultaneous, separate or sequential use in the prevention and/or treatment of viral infection by an influenza virus.
